# EUROPEAN PATENT SPECIFICATION

(11) **EP 4 387 589 B1**
(45) Date of publication and mention of the grant of the patent: **09.12.2026**
(21) Application number: 21800075.0
(22) Date of filing: 06.10.2021
(51) Int. Cl.: A61K 9/00, A61K 9/107, A61K 47/44, A61K 38/13, A61P 27/02, A61K 47/32

(54) **STABLE CYCLOSPORINE OPHTHALMIC FORMULATION AND MANUFACTURING PROCESS THEREOF**
STABILE OPHTHALMISCHE CYCLOSPORINFORMULIERUNG UND HERSTELLUNGSVERFAHREN DAFÜR
FORMULATION OPHTALMIQUE DE CYCLOSPORINE STABLE ET SON PROCÉDÉ DE FABRICATION

(30) Priority: 20.08.2021 IN 202121037917
(43) Date of publication of application: 26.06.2024
(73) Proprietor: Sun Pharmaceutical Industries Limited, Mumbai Maharashtra 400 063 (IN)
(72) Inventor: KHOPADE, Ajay Jaysingh, Vadodara, Gujarat 390020 (IN); HALDER, Arindam, Vadodara, Gujarat 390020 (IN)
(74) Representative: Nederlandsch Octrooibureau
(86) International application number: PCT/IB2021/059176
(87) International publication number: WO 2021/224901

(56) References cited:
- US-A1- 2014 057 854
- US-A1- 2019 060 397
- US-B2- 9 937 225
- GOLDBERG DAMIEN F ET AL: "A Phase 3, Randomized, Double-Masked Study of OTX-101 Ophthalmic Solution 0.09% in the Treatment of Dry Eye Disease", OPHTHALMOLOGY, ELSEVIER, AMSTERDAM, NL, vol. 126, no. 9, 6 April 2019 (2019-04-06), pages 1230 - 1237, XP085784272, ISSN: 0161-6420, [retrieved on 20190406], DOI: 10.1016/J.OPHTHA.2019.03.050
- LALU LIDA ET AL: "Novel nanosystems for the treatment of ocular inflammation: Current paradigms and future research directions", JOURNAL OF CONTROLLED RELEASE, ELSEVIER, AMSTERDAM, NL, vol. 268, 27 July 2017 (2017-07-27), pages 19 - 39, XP085300163, ISSN: 0168-3659, DOI: 10.1016/J.JCONREL.2017.07.035
- MANDAL ABHIRUP ET AL: "Ocular Pharmacokinetics of a Topical Ophthalmic Nanomicellar Solution of Cyclosporine (Cequa ) for Dry Eye Disease", PHARMACEUTICAL RESEARCH, SPRINGER US, NEW YORK, vol. 36, no. 2, 7 January 2019 (2019-01-07), pages 1 - 21, XP036668339, ISSN: 0724-8741, [retrieved on 20190107], DOI: 10.1007/S11095-018-2556-5
- CHAZOTTE, B.: "Labeling Golgi with Fluorescent Ceramides", COLD SPRING HARBOR PROTOCOLS, vol. 2012, no. 8, 1 August 2012 (2012-08-01), pages 913 - 916, DOI: 10.1101/pdb.prot070599
- KYMIONIS GEORGE, KYMIONIS GEORGE: "Treatment of chronic dry eye: focus on cyclosporine", CLINICAL OPHTHALMOLOGY, DOVE MEDICAL PRESS LTD., NZ, vol. 2, no. 4, 1 December 2008 (2008-12-01), NZ , pages 829 - 836, XP055891709, ISSN: 1177-5467, DOI: 10.2147/OPTH.S1409

## Description

### FIELD OF THE INVENTION

The present invention relates to a stable nanomicellar ophthalmic solution comprising cyclosporine and a method of preparing the nanomicellar solution. The present invention further relates to the stable nanomicellar solution comprising cyclosporine form with characteristic XRD peaks at 2-theta (deg.) 6.9, 7.8, 9.4 and 15.9 or amorphous cyclosporine. The present invention also relates to use of this stable nanomicellar ophthalmic solution in dry eye.

### BACKGROUND OF THE INVENTION

Cyclosporine nanomicellar ophthalmic solutions are generally disclosed in U.S. Patent No. 10,918,694, wherein the ophthalmic solution comprises comprising 0.087-0.093 wt % cyclosporine, a polyoxyl lipid or fatty acid and a polyalkoxylated alcohol. Preferably, it comprises 0.087-0.093 wt % cyclosporine, 0.5-5% of one or more selected from the group consisting of HCO-40, HCO-60, HCO-80 and HCO-100; and about 0.01-0.1% octoxynol-40. Further, it discloses methods of preparing such cyclosporine solutions. The method includes dissolution of cyclosporine in polyoxyl castor oil such as hydrogenated castor oil and a polyalkoxylated alcohol such as octoxynol at 60°C prior to addition in an aqueous phase. Specifically, the preparation method of ophthalmic solution consists of the following steps: HCO-40 is melted in a flask heated to about 60°C with stirring. When liquefied, the required amount of cyclosporine is added and mixed until dissolved and uniform. Then, octoxynol-40 is heated to about 60°C and when liquefied, is added to the cyclosporine HCO-40 mixture. Water for injection at about 25°C is charged into the flask containing the dissolved cyclosporine and stirred until dissolved. Other excipients are then added, such as sodium chloride and phosphate buffer and then PVP-K90 and mixed until dissolved and brought to the final volume with water for injection. However, current methods have a problem in that when cyclosporine is dissolved in the polyoxyl castor oil, such as HCO-40, due to the difference in solubility and stability of different forms of cyclosporine, there may be a difference in stability of different batches during manufacturing. The result is that some batches are not stable and the cyclosporine precipitates out of solution.

Further, cyclosporine in different forms shows different solubility and stability. Cyclosporine with characteristic XRD peaks at 2-theta (deg.) 6.9, 7.8, 9.4 and 15.9 is the most soluble form of cyclosporine and is useful in the preparation of solution formulations, however this is not the most stable form and it may convert to the less soluble forms of cyclosporine, thus affecting the stability of the solution. This form may convert to a more stable and less soluble cyclosporine with characteristic XRD peaks at 2-theta (deg.) 7.4, 8.7, 14.4 and 17.5 during dissolution of cyclosporine in surfactants at 55-60°C. Similarly, it may change to another less soluble form of cyclosporine with characteristic XRD peaks at 2-theta (deg.) 8.5, 9.3, 11.6 and 20.3. Further, the amorphous form, which is also one of the more soluble forms and useful in the preparation of solution formulations, may also recrystallize to these two less soluble forms. Such a conversion is facilitated by several factors not limited to moisture, water, solvent temperature and so on. This conversion also depends on stresses like temperature, long storage at higher temperature, and the like. The process needs modification depending on the form of cyclosporine used, such that the amorphous form and the form with characteristic XRD peaks at 2-theta (deg.) 6.9, 7.8, 9.4 and 15.9 needs wetting followed by complete dissolution at the temperature up to 70°C. However, if the less soluble forms are used, temperatures as high as 130°C are needed. Further, during the manufacturing steps, due to the instability and conversions, the solution is to be manufactured with tight control of API specification, process temperature and time to avoid interconversion of forms. It is rather, difficult to identify to what extent this conversion has taken place before completing the manufacturing process to get the nanomicellar cyclosporine. This leads to a formation of seeds within the composition either at the initial stage of the process or during storage at higher temperatures, which later crystallized out from the drug product rendering the product unuseful. The soft mesophasic or liquid crystalline form of cyclosporine formed as intermediate in the non-aqueous phase may be responsible for such instability. US 2019/0060397 A1 discloses a process of preparing an ophthalmic topical formulation of cyclosporine, said method comprising the steps of: (1) melting the required amount of hydrogenated 40 polyoxyl castor oil, (2) slowly adding cyclosporine to step (1) and substantially homogenizing the mixture, (3) adding octoxynol-40 alcohol to step (2) and continue stirring until a uniform homogeneous solution is obtained, (4) adding phosphate buffer and sodium chloride to the solution obtained from step (3) and continue stirring to achieve a good dissolution, (5) adding required amount of povidone to the solution of above step, (6) adjusting the pH of the solution if required, and making up the final volume with water for injection; and (7) aseptically filtering and filling the solution into unit dose vials. Goldberg Damien F. et al. (Ophthalmology, Vol. 126, 2019, pages 1230-1237) report on a phase 3, randomized, double-masked study of OTX-101 ophthalmic solution 0.09% in the treatment of dry eye disease.

Thus, there is a need for a stable formulation and method of its preparation to prevent conversion to less soluble forms of cyclosporine during the manufacturing process and during storage. The present invention discloses a stable nanomicellar ophthalmic formulation and an improved method of making such stable formulation. The method for making the formulation results in a stable formulation irrespective of any form of cyclosporin being used in the formulation. The method does not lead to conversion of one form to another. More specifically, does not lead to conversion of the soluble form of cyclosporine to the less soluble forms of cyclosporine and further prevents precipitation of cyclosporine in the formulation on long-term stability.

### SUMMARY OF THE INVENTION

One of the objectives of the present invention, according to some embodiments, a method of making a stable nanomicellar ophthalmic formulation comprising:
cyclosporine, a polyoxyl lipid or fatty acid, and a polyalkoxylated alcohol, wherein the ophthalmic formulation is made by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40; and
c) mixing the resulting mixture with the aqueous vehicle.

The present inventors have surprisingly found that the solution stability of cyclosporine A at 35°C-40°C was higher than that of 55°C-60°C and thus, reducing the temperature to 35°C-40°C overcomes the above mentioned stability concerns of the formulation and provides for a more stable formulation.

In another aspect, the present invention is drawn to a stable nanomicellar ophthalmic formulation comprising:
cyclosporine, hydrogenated 40 polyoxyl castor oil, octoxynol-40, and an aqueous vehicle, wherein the ophthalmic formulation is made by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40; and
c) mixing the resulting mixture with the aqueous vehicle.

In one aspect, mixing the aqueous vehicle at a temperature of at 35°C±2°C. In another aspect, mixing the aqueous vehicle at a temperature of 55±2°C.

In one aspect, the present invention provides a stable nanomicellar ophthalmic formulation comprising cyclosporine form with characteristic XRD peaks at 2-theta (deg.) 6.9, 7.8, 9.4 and 15.9.

In another aspect, the present invention provides a stable nanomicellar ophthalmic formulation comprising an amorphous form of cyclosporine.

In another aspect, a method of making a stable nanomicellar ophthalmic formulation comprising: cyclosporine, hydrogenated 40 polyoxyl castor oil, octoxynol-40, and an aqueous vehicle,
wherein the ophthalmic formulation is made by a method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40; and
e) mixing the resulting mixture with the aqueous vehicle.

In another embodiment, it provides a stable nanomicellar ophthalmic formulation comprising:
cyclosporine, hydrogenated 40 polyoxyl castor oil, octoxynol-40, and an aqueous vehicle, wherein the ophthalmic formulation is made by a method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40; and
e) mixing the resulting mixture with the aqueous vehicle.
It was surprisingly found that the on keeping the mixture under vacuum, the bubbles are dragged up and accumulate on the surface from where bubbles were gradually removed and the bottom portion is cleared up. So dissolution under vacuum or removal of foams intermittently during dissolution leads to faster dissolution and may not require the lowering of the temperature of the mixture. Thus, overcomes the above mentioned stability concerns of the formulation and provides for a more stable formulation.

In one aspect, mixing the aqueous vehicle at a temperature of at 35°C±2°C. In another aspect, mixing the aqueous vehicle at a temperature of 55±2°C.

In one aspect, the present invention provides a stable nanomicellar ophthalmic formulation comprising cyclosporine form with characteristic XRD peaks at 2-theta (deg.) 6.9, 7.8, 9.4 and 15.9.

In another aspect, the present invention provides a stable nanomicellar ophthalmic formulation comprising an amorphous form of cyclosporine.

In yet another aspect, the present invention provides a stable nanomicellar ophthalmic formulation comprising:
cyclosporine, hydrogenated 40 polyoxyl castor oil, octoxynol-40, and an aqueous vehicle, wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of at 127-130°C,
wherein, cyclosporine is present in a form having characteristic XRD peaks at 2-theta (deg.) 7.4, 8.7, 14.4 and 17.5.

In yet another aspect, the present invention provides a stable nanomicellar ophthalmic formulation comprising:
cyclosporine, hydrogenated 40 polyoxyl castor oil, octoxynol-40, and an aqueous vehicle, wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of 127-130°C,
wherein, cyclosporine is present in a form with characteristic XRD peaks at 2-theta (deg.) 8.5, 9.3, 11.6 and 20.3.

In a preferred aspect, the present invention provides a method of making a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40; and
c) mixing the resulting mixture with the aqueous vehicle.

In one aspect, the aqueous vehicle is mixed at a temperature of at 35°C±2°C. In another aspect, the aqueous vehicle is mixed at a temperature of 55±2°C.

In a preferred aspect, the present invention also provides a stable nanomicellar ophthalmic formulation prepared by a method as described above.

In a preferred aspect, the present invention provides a method of making a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40; and
e) mixing the resulting mixture with the aqueous vehicle.

In one aspect, the aqueous vehicle is mixed at a temperature of at 35°C±2°C. In another aspect, the aqueous vehicle is mixed at a temperature of 55±2°C.

In a preferred aspect, the present invention also provides a stable nanomicellar ophthalmic formulation prepared by a method as described above.

In one aspect, the stable nanomicellar ophthalmic formulation further comprises:
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection.

In one aspect, the present invention provides a stable nanomicellar ophthalmic formulation, wherein the pH of the formulation is about 5.0 to 8.0. More preferably, the pH of the formulation is about 6.5 to 7.2.

Further, the present invention provides a stable nanomicellar ophthalmic formulation wherein the osmolality of the formulation is between about 150 to about 200 mOsmol/kg.

In another aspect, the present invention provides a stable nanomicellar ophthalmic formulation comprising cyclosporine form with characteristic XRD peaks at 2-theta (deg.) 6.9, 7.8, 9.4 and 15.9.

In yet another aspect, the present invention provides a stable nanomicellar ophthalmic formulation comprising an amorphous form of cyclosporine.

In another aspect, the present invention provides a stable nanomicellar ophthalmic formulation, wherein the formulation is substantially free of a cyclosporine form with characteristic XRD peaks at 2-theta (deg.) 7.4, 8.7, 14.4 and 17.5.

In another aspect, the present invention provides a stable nanomicellar ophthalmic formulation, wherein the formulation is substantially free of a cyclosporine form with characteristic XRD peaks at 2-theta (deg.) 8.5, 9.3, 11.6 and 20.3.

In another aspect, the present invention provides a stable nanomicellar ophthalmic formulation, prepared by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40; and
c) then mixing the resulting mixture with an aqueous vehicle at 35°C±2°C, wherein the mixture A is lowered to a temperature of 35°C±2°C in less than 65 minutes. Preferably, the mixture A is lowered to a temperature of 35°C±2°C in 40-50 minutes.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of to 35°C±2°C and stirring for 60-70 minutes. Preferably, stirring the mixture for 60±5 minutes at a temperature of 35°C±2°C.

In yet another aspect, the present invention provides a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of at 127-130°C,
wherein, cyclosporine is present in a form having characteristic XRD peaks at 2-theta (deg.) 7.4, 8.7, 14.4 and 17.5.

In yet another aspect, drawn to a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of 127-130°C,
wherein, cyclosporine is present in a form with characteristic XRD peaks at 2-theta (deg.) 8.5, 9.3, 11.6 and 20.3.

In yet another aspect, the present invention provides a method of making a stable nanomicellar ophthalmic formulation comprising:
0.09 wt % cyclosporine,
about 1.0 wt % hydrogenated 40 polyoxyl castor oil,
about 0.05 wt % octoxynol-40,
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection,
wherein the method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40 to form an API mixture;
c) adding API mixture to Water for Injection (WFI);
d) adding remaining excipients to step (c) in the order of sodium phosphate monobasic, then sodium phosphate dibasic, then sodium chloride, and then polyvinylpyrrolidone;
e) adjusting the pH to 6.5 to 7.2 and bringing to the final volume with WFI.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of the mixture A to 35°C±2°C in less than 65 minutes. Preferably, lowering the temperature of the mixture A to 35°C±2°C in 40-50 minutes.

In another aspect, stirring the mixture of step (b) for 60-70 minutes. Preferably, stirring the mixture for 60±5 minutes at a temperature of 35°C±2°C.

In one aspect, mixing the API mixture to WFI at a temperature of 35°C±2°C. In another aspect, mixing the API mixture to WFI at a temperature of 55±2°C.

In yet another aspect, the present invention provides a method of making a stable nanomicellar ophthalmic formulation comprising:
0.09 wt % cyclosporine,
about 1.0 wt % hydrogenated 40 polyoxyl castor oil,
about 0.05 wt % octoxynol-40,
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection,
wherein the method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40;
e) adding API mixture to Water for Injection (WFI);
f) adding remaining excipients to step (c) in the order of sodium phosphate monobasic, then sodium phosphate dibasic, then sodium chloride, and then polyvinylpyrrolidone; and
g) adjusting the pH to 6.5 to 7.2 and bringing to the final volume with WFI.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of the mixture A to 35°C±2°C in less than 65 minutes. Preferably, lowering the temperature of the mixture A to 35°C±2°C in 40-50 minutes.

In one aspect, mixing the API mixture to WFI at a temperature of 35°C±2°C. In another aspect, mixing the API mixture to WFI at a temperature of 55±2°C.

In a more preferred aspect, the present invention provides for a stable nanomicellar ophthalmic formulation prepared by any of the method as described above.

### BRIEF DESCRIPTION OF THE DRAWINGS

Figure 1(a) and Figure 1(b) depict cyclosporine nanomicellar ophthalmic formulations prepared by methods described in Example 1(a) and Example 1(b), respectively. Figure 1(a) discloses that no particles were observed, while Figure 1(b) discloses that particles were observed on stability.
Figure 2 depicts characteristic X-ray powder diffraction (XRPD) patterns of cyclosporine with a cyclosporine form with characteristic XRD peaks at 2-theta (deg.) 6.9, 7.8, 9.4 and 15.9 (A), a cyclosporine form with characteristic XRD peaks at 2-theta (deg.) 7.4, 8.7, 14.4 and 17.5 (B), a cyclosporine form with characteristic XRD peaks at 2-theta (deg.) 8.5, 9.3, 11.6 and 20.3 (C), and an amorphous form of cyclosporine.
Figures 3(a) to 3(d) depict the solubility behavior of different cyclosporine forms at 55°C. Figure 3(a) depicts the solubility behavior of CsA form with characteristic XRD peaks at 2-theta (deg.) 6.9, 7.8, 9.4 and 15.9. Figure 3(b) depicts solubility behavior of a CsA form with characteristic XRD peaks at 2-theta (deg.) 7.4, 8.7, 14.4 and 17.5. Figure 3(c) depicts solubility behaviour of a CsA form with characteristic XRD peaks at 2-theta (deg.) 8.5, 9.3, 11.6 and 20.3, and Figure 3(d) depicts solubility behaviour of amorphous CsA.
Figure 4 depicts X-ray powder diffraction (XRPD) patterns of a precipitated cyclosporine from solution prepared when cyclosporine is dissolved in Kolliphore RH 40 at higher exposure and temperature.
Figure 5 depicts X-ray powder diffraction (XRPD) patterns of a precipitated cyclosporine when cyclosporine is dissolved in Kolliphore RH 40 kept it for a longer time till the precipitation occurred.

### DETAILED DESCRIPTION OF THE INVENTION

One embodiment of the present disclosure is a method of making a stable nanomicellar ophthalmic formulation comprising:
0.09 wt % cyclosporine, about 1.0 wt % hydrogenated 40 polyoxyl castor oil, about 0.05 wt % octoxynol-40, about 0.20-0.550 wt % sodium phosphate monobasic, about 0.23-0.465 wt % sodium phosphate dibasic, about 0.05 wt % sodium chloride, about 0.3 wt % povidone, sodium hydroxide/hydrochloric acid, and water for injection,
wherein the ophthalmic formulation is made by a method comprising the steps of:
   a) mixing the cyclosporine with the polyoxyl lipid or fatty acid at a temperature of 55°C or above to form a mixture A; and
   b) lowering the temperature of mixture A to a temperature not higher than 40°C prior to the complete dissolution of the cyclosporine.

Another embodiment of the present disclosure is a table nanomicellar ophthalmic formulation prepared by the method as described before, wherein the formulation is substantially free of (i) a cyclosporine form with characteristic XRD peaks at 2-theta (deg.) 7.4, 8.7, 14.4 and 17.5 or (ii) a cyclosporine form with characteristic XRD peaks at 2-theta (deg.) 8.5, 9.3, 11.6 and 20.3.

Preferably, mixing the cyclosporine with the polyoxyl lipid or fatty acid is done at a temperature of 55°C-60°C to form a mixture A. Preferably, mixture A is lowered to a temperature of 35°C- 40°C prior to the complete dissolution of the cyclosporine. More preferably, mixing the cyclosporine with the polyoxyl lipid or fatty acid is done at a temperature of 55°C±2°C to form a mixture A; and mixture A is lowered to a temperature not higher than 35°C±2°C prior to the complete dissolution of the cyclosporine. Further, the method comprises adding the polyalkoxylated alcohol and then mixing the resulting mixture with an aqueous vehicle at 35°C±2°C. In another aspect, the resulting mixture with an aqueous vehicle at a temperature of 55±2°C.

Another embodiment of the present disclosure is a method of making a stable nanomicellar ophthalmic formulation comprising:
cyclosporine, a polyoxyl lipid or fatty acid, and a polyalkoxylated alcohol, and an aqueous vehicle,
wherein the ophthalmic formulation is made by a method comprising the steps of:
   a) mixing the cyclosporine with the polyoxyl lipid or fatty acid at a temperature of 55°C±2°C or above to form a mixture A;
   b) keeping mixture A under vacuum to remove foam;
   c) optionally, lowering the temperature of mixture A to a temperature of 35°C±2°C prior to the complete dissolution of the cyclosporine, adding polyalkoxylated alcohol; and
   d) mixing the resulting mixture with the aqueous vehicle.

In one aspect, the aqueous vehicle is mixed at a temperature of at 35°C±2°C. In another aspect, the aqueous vehicle is mixed at a temperature of 55±2°C.

It was found that during the dissolution of hydrophobic molecule like cyclosporine A (CsA) in polyoxyl lipid such as polyoxyl hydrogenated castor oil, it was found that air was entrapped in the bulk in a form of bubbles and creates foam. This happens irrespective of temperature during the drug dissolution process, either 55°C or even when the temperature was reduced to 35°C. This may delay the wetting and dissolution of cyclosporine as this entrapped air may make a boundary between cyclosporine particles and water. It was surprisingly found that the on keeping the mixture under vacuum, the bubbles are dragged up and accumulate on the surface from where bubbles were gradually removed and the bottom portion is cleared up. So dissolution under vacuum or removal of foams intermittently during dissolution leads to faster dissolution and may not require the lowering of the temperature of the mixture.

In one aspect, the method of making a stable nanomicellar ophthalmic formulation comprises cyclosporine form with characteristic XRD peaks at 2-theta (deg.) 6.9, 7.8, 9.4 and 15.9.

In another aspect, the method of making a stable nanomicellar ophthalmic formulation comprises an amorphous form of cyclosporine.

Another embodiment of the present disclosure is a method of making a stable nanomicellar ophthalmic formulation comprising cyclosporine, a polyoxyl lipid or fatty acid, a polyalkoxylated alcohol, and an aqueous vehicle, wherein the ophthalmic formulation is made by a method comprising the steps of:
a) mixing the cyclosporine with a polyoxyl lipid or fatty acid at a temperature of 127-130°C until completely dissolved to form a mixture A;
b) adding the polyalkoxylated alcohol to mixture A at 127-130°C; and
c) mixing the resulting mixture with the aqueous vehicle at a temperature of at 127-130°C,
wherein the cyclosporine is present in a form having characteristic XRD peaks at 2-theta (deg.) 7.4, 8.7, 14.4 and 17.5.

In yet another aspect, the cyclosporine is present in a form with characteristic XRD peaks at 2-theta (deg.) 8.5, 9.3, 11.6 and 20.3.

Another embodiment of the present disclosure is a stable nanomicellar ophthalmic formulation comprising cyclosporine, a polyoxyl lipid or fatty acid, and a polyalkoxylated alcohol, wherein the ophthalmic formulation is made by a method comprising the steps of:
a) mixing the cyclosporine with the polyoxyl lipid or fatty acid at a temperature of 55°C or above to form a mixture A; and
b) lowering the temperature of mixture A to a temperature not higher than 40°C prior to the complete dissolution of the cyclosporine.

Preferably, mixing the cyclosporine with the polyoxyl lipid or fatty acid is done at a temperature of 55°C-60°C to form a mixture A. Preferably, mixture A is lowered to a temperature 35°C- 40°C prior to the complete dissolution of the cyclosporine. More preferably, mixing the cyclosporine with the polyoxyl lipid is done at a temperature of 55°C±2°C to form a mixture A; and mixture A is lowered to a temperature not higher than 35°C±2°C prior to the complete dissolution of the cyclosporine. Further, the method comprises adding polyalkoxylated alcohol and then mixing the resulting mixture with an aqueous vehicle at 35°C±2°C. In another aspect, the resulting mixture is mixed with the aqueous vehicle at a temperature of 55±2°C.

In one aspect, the present invention provides a stable nanomicellar ophthalmic formulation comprising cyclosporine form with characteristic XRD peaks at 2-theta (deg.) 6.9, 7.8, 9.4 and 15.9.

In another aspect, the present invention provides a stable nanomicellar ophthalmic formulation comprising an amorphous form of cyclosporine.

In another aspect, the present invention provides a stable nanomicellar ophthalmic formulation comprising: cyclosporine, a polyoxyl lipid or fatty acid, and a polyalkoxylated alcohol, and an aqueous vehicle,
wherein the ophthalmic formulation is made by a method comprising the steps of:
a) mixing the cyclosporine with the polyoxyl lipid or fatty acid at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine, adding polyalkoxylated alcohol; and
d) mixing the resulting mixture with the aqueous vehicle.

In one aspect, the aqueous vehicle is mixed at a temperature of at 35°C±2°C. In another aspect, the aqueous vehicle is mixed at a temperature of 55±2°C.

In one aspect, the stable nanomicellar ophthalmic formulation comprises cyclosporine form with characteristic XRD peaks at 2-theta (deg.) 6.9, 7.8, 9.4 and 15.9.

In another aspect, the stable nanomicellar ophthalmic formulation comprises an amorphous form of cyclosporine.

In yet another aspect, the present invention provides a stable nanomicellar ophthalmic formulation comprising:
cyclosporine, a polyoxyl lipid or fatty acid, a polyalkoxylated alcohol, and an aqueous vehicle wherein the ophthalmic formulation is made by a method comprising the steps of:
a) mixing the cyclosporine with a polyoxyl lipid or fatty acid at a temperature of 127-130°C until completely dissolved to form a mixture A;
b) adding the polyalkoxylated alcohol to mixture A at 127-130°C; and
c) mixing the resulting mixture with the aqueous vehicle at a temperature of at 127-130°C,
wherein the cyclosporine is present in a form having characteristic XRD peaks at 2-theta (deg.) 7.4, 8.7, 14.4 and 17.5.

In yet another aspect, the cyclosporine is present in a form with characteristic XRD peaks at 2-theta (deg.) 8.5, 9.3, 11.6 and 20.3.

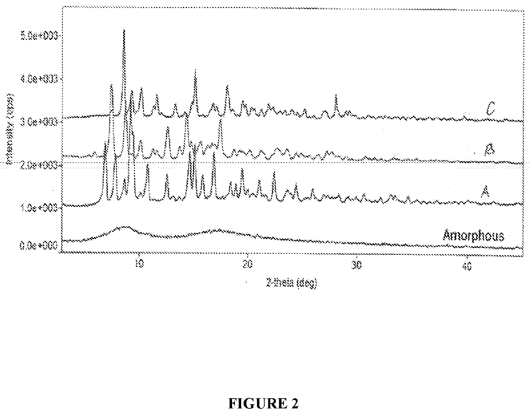

Another embodiment of the present disclosure is a method of making a stable nanomicellar ophthalmic formulation comprising a cyclosporine, a polyoxyl lipid or fatty acid, and a polyalkoxylated alcohol, wherein the ophthalmic formulation is made by a method comprising the steps of: mixing the cyclosporine with the polyoxyl lipid or fatty acid at a temperature of 55°C or above to form a mixture A; and preventing the formation of the cyclosporine form with characteristic XRD peaks at 2-theta (deg.) 7.4, 8.7, 14.4 and 17.5 (B in Figure 2) by altering the temperature of mixture A prior to the complete dissolution of the cyclosporine.

Preferably, mixing the cyclosporine with the polyoxyl lipid or fatty acid is done at a temperature of 55°C-60°C to form a mixture A. Preferably, mixture A is lowered to a temperature 35°C- 40°C prior to the complete dissolution of the cyclosporine. More preferably, mixing the cyclosporine with the polyoxyl lipid is done at a temperature of 55°C±2°C to form a mixture A; and mixture A is lowered to a temperature not higher than 35°C±2°C prior to the complete dissolution of the cyclosporine. In one aspect, the method of making a stable nanomicellar ophthalmic formulation comprises cyclosporine with characteristic XRD peaks at 2-theta (deg.) 6.9, 7.8, 9.4 and 15.9 (A in Figure 2). In another aspect, the method of making a stable nanomicellar ophthalmic formulation comprises an amorphous form of cyclosporine. Further, the method comprises adding the polyalkoxylated alcohol and then mixing the resulting mixture with an aqueous vehicle at 35°C±2°C. In another aspect, the resulting mixture is mixed with the aqueous vehicle at a temperature of 55±2°C.

Another embodiment of the present disclosure is a method of making a stable nanomicellar ophthalmic formulation comprising a cyclosporine, a polyoxyl lipid or fatty acid, and a polyalkoxylated alcohol, wherein the ophthalmic formulation is made by a method comprising the steps of: mixing the cyclosporine with the polyoxyl lipid or fatty acid at a temperature of 55°C or above to form a mixture A; and applying vacuum to the mixture to prevent the formation of the cyclosporine form with characteristic XRD peaks at 2-theta (deg.) 7.4, 8.7, 14.4 and 17.5 (B in Figure 2).

Preferably, mixing the cyclosporine with the polyoxyl lipid or fatty acid is done at a temperature of 55°C-60°C to form a mixture A. Optionally, the mixture A is lowered to a temperature 35°C- 40°C prior to the complete dissolution of the cyclosporine. More preferably, mixing the cyclosporine with the polyoxyl lipid is done at a temperature of 55°C±2°C to form a mixture A; optionally lowering to a temperature not higher than 35°C±2°C prior to the complete dissolution of the cyclosporine. Further, the method comprises adding the polyalkoxylated alcohol and then mixing the resulting mixture with an aqueous vehicle at 35°C±2°C. In another aspect, the resulting mixture is mixed with the aqueous vehicle at a temperature of 55±2°C.

Another embodiment of the present disclosure is a method of making a stable nanomicellar ophthalmic formulation comprising a cyclosporine, a polyoxyl lipid or fatty acid, and a polyalkoxylated alcohol, wherein the ophthalmic formulation is made by a method comprising the steps of: mixing the cyclosporine with the polyoxyl lipid or fatty acid at a temperature of 55°C or above to form a mixture A; and preventing the formation of the cyclosporine form with characteristic XRD peaks at 2-theta (deg.) 8.5, 9.3, 11.6 and 20.3 (C in Figure 2) by altering the temperature of mixture A prior to the complete dissolution of the cyclosporine.

Preferably, mixing the cyclosporine with the polyoxyl lipid or fatty acid is done at a temperature of 55°C-60°C to form a mixture A. Further, mixture A is lowered to a temperature 35°C- 40°C prior to the complete dissolution of the cyclosporine. More preferably, mixing the cyclosporine with the polyoxyl lipid is done at a temperature of 55°C±2°C to form a mixture A; and mixture A is then lowered to a temperature not higher than 35°C±2°C prior to the complete dissolution of the cyclosporine. In one aspect, the method of making a stable nanomicellar ophthalmic formulation comprises cyclosporine with characteristic XRD peaks at 2-theta (deg.) 6.9, 7.8, 9.4 and 15.9 (A in Figure 2). In another aspect, the method of making a stable nanomicellar ophthalmic formulation comprises an amorphous form of cyclosporine. Further, the method comprises adding the polyalkoxylated alcohol and then mixing the resulting mixture with an aqueous vehicle at 35°C±2°C. In another aspect, the resulting mixture is mixed with the aqueous vehicle at a temperature of 55±2°C.

Another embodiment of the present disclosure is a method of making a stable nanomicellar ophthalmic formulation comprising a cyclosporine, a polyoxyl lipid or fatty acid, and a polyalkoxylated alcohol, wherein the ophthalmic formulation is made by a method comprising the steps of: mixing the cyclosporine with the polyoxyl lipid or fatty acid at a temperature of 55°C or above to form a mixture A; and applying vacuum to the mixture to prevent the formation of the cyclosporine form with characteristic XRD peaks at 2-theta (deg.) 8.5, 9.3, 11.6 and 20.3 (C in Figure 2).

Preferably, mixing the cyclosporine with the polyoxyl lipid or fatty acid is done at a temperature of 55°C-60°C to form a mixture A. Optionally, the mixture A is lowered to a temperature 35°C- 40°C prior to the complete dissolution of the cyclosporine. More preferably, mixing the cyclosporine with the polyoxyl lipid is done at a temperature of 55°C±2°C to form a mixture A; optionally lowering to a temperature not higher than 35°C±2°C prior to the complete dissolution of the cyclosporine. Further, the method comprises adding the polyalkoxylated alcohol and then mixing the resulting mixture with an aqueous vehicle at 35°C±2°C. In another aspect, the resulting mixture is mixed with the aqueous vehicle at a temperature of 55±2°C.

Another embodiment of the present disclosure is a stable nanomicellar ophthalmic formulation comprising cyclosporine, a polyoxyl lipid or fatty acid, and a polyalkoxylated alcohol, wherein the formulation is a solution; and the formulation exhibits stability at room temperature (20-25°C) for 6 to at least 24 months. Typically, the ophthalmic formulations are stable when maintained at room temperature for at least 6 months, at least 8 months, at least 10 months, at least 12 months, at least 14 months, at least 16 months, at least 18 months, at least 20 months and at least 24 months.

In another embodiment, the present disclosure provides a stable nanomicellar ophthalmic formulation that exhibits stability at 2°C to 8°C for 6 to at least 24 months. Typically, the ophthalmic formulations are stable when maintained at 2°C to 8°C for at least 6 months, at least 8 months, at least 10 months, at least 12 months, at least 14 months, at least 16 months, at least 18 months, at least 20 months and at least 24 months.

Materials useful in the formulations of the present disclosure include, but are not limited to, those disclosed in U.S. Patent No. 10,918,694.

As they are used here, the terms "cyclosporin", "cyclosporine", "cyclosporine A", or "CsA" may be used interchangeably and includes pharmaceutically acceptable salts of the same.

As used herein in connection with numerical values, the terms "approximately" and "about" mean +/-10% of the indicated value, including the indicated value.

As used herein, the term "substantially free" refers to an amount of 10% or less of the indicated substance, such as another form, preferably 8%, 5%, 4%, 3%, 2%, 1%, 0.5%, or less of another form.

As used herein, the term "polyoxyl lipid or fatty acid" refers to mono- and diesters of lipids or fatty acids and polyoxyethylene diols. Polyoxyl lipids or fatty acids may be numbered ("n") according to the average polymer length of the oxyethylene units (e.g., 40, 60, 80, 100) as is well understood in the art. The term "n≥40 polyoxyl lipid" means that the polyoxyl lipid or fatty acid has an average oxyethylene polymer length equal to or greater than 40 units. Stearate hydrogenated castor oil and castor oil are common lipids/fatty acids commercially available as polyoxyl lipids or fatty acid, however, it is understood that any lipid or fatty acid could polyoxylated to become a polyoxyl lipid or fatty acid as contemplated herein. Examples of polyoxyl lipid or fatty acids include without limitation hydrogenated polyoxyl castor oil such as HCO-40, HCO-60, HCO-80, HCO-100, or polyoxyl 40 stearate, polyoxyl 35 castor oil.

As used herein, the term "micelle" or "nanomicelle" refers to an aggregate (or cluster) of surfactant molecules. Micelles only form when the concentration of surfactant is greater than the critical micelle concentration (CMC). Surfactants are chemicals that are amphipathic, which means that they contain both hydrophobic and hydrophilic groups. Micelles can exist in different shapes, including spherical, cylindrical, and discoidal. A micelle comprising at least two different molecular species is a mixed micelle. The in some embodiments, ophthalmic compositions of the present disclosure include an aqueous, clear, mixed micellar solution.

In some embodiments the formulations include, but are not limited to, nanomicelles, as disclosed in U.S. Patent No. 10,918,694. For example, ophthalmic compositions can be administered topically to the eye as biocompatible, aqueous, clear mixed micellar solutions. The compositions have the drugs incorporated and/or encapsulated in micelles which are dispersed in an aqueous medium.

In some aspects of embodiments, the polyoxyl lipid or fatty acid is a polyoxyl castor oil. In some embodiments, the polyoxyl lipid or fatty acid is one or more selected from hydrogenated polyoxyl castor oil such as HCO-40, HCO-60, HCO-80 or HCO-100. In some embodiments the polyoxyl lipid or fatty acid (such as a polyoxyl castor oil such as HCO-60, HCO-80 or HCO-100) is present between 0.5 and 2%, or 0.7 and 2%, or 1 and 6%; or 2 and 6%; or 2 and 6%; or 3 and 6%; or 4 and 6%; or 2 and 5%; or 3 and 5%; or 3 and 5%; or 2 and 6%; or about 4%; or greater than 0.7%; or greater than 1%, or greater than 1.5%; or greater than 2%; or greater than 3%; or greater than 4% by weight of the formulation. In some embodiments, the polyoxyl lipid is HCO-40. In some aspects of embodiments, the polyoxyl lipid is HCO-60. In some embodiments, the polyoxyl lipid is HCO-80. In some embodiments, the polyoxyl lipid is HCO-100.

In some aspects of embodiments, the formulation includes a polyalkoxylated alcohol. In some embodiments, the polyalkoxylated alcohol is octoxynol-40. In some aspects of embodiments, the formulation includes a polyalkoxylated alcohol (such as octoxynol-40) present between 0.002 and 4%; or between 0.005 and 3%; or between 0.005 and 2%; or between 0.005 and 1%; or between 0.005 and 0.5%; or between 0.005 and 0.1%; or between 0.005 and 0.05%; or between 0.008 and 0.02%; or between 0.01 and 0.1%; or between 0.02 and 0.08%; or between 0.005 and 0.08%; or about 0.05%, or about 0.01% by weight of the formulation.

One embodiment of the present disclosure is a stable nanomicellar ophthalmic formulation. The ophthalmic formulation comprises cyclosporine, a hydrogenated polyoxyl castor oil, and octoxynol-40. Preferably, the hydrogenated polyoxyl castor oil is hydrogenated 40 polyoxyl castor oil (HCO-40). Preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising cyclosporine, a hydrogenated polyoxyl castor oil, and octoxynol-40, wherein the ophthalmic formulation is made by a method comprising the steps of:
a) mixing the cyclosporine with a hydrogenated polyoxyl castor oil at a temperature of 55°C or above to form a mixture A; and
b) lowering the temperature of mixture A to a temperature not higher than 40°C prior to the complete dissolution of the cyclosporine.

Preferably, mixing the cyclosporine with a hydrogenated polyoxyl castor oil is done at a temperature of 55°C-60°C to form a mixture A. Preferably, mixture A is lowered to a temperature 35°C- 40°C prior to the complete dissolution of the cyclosporine. More preferably, mixing the cyclosporine with the hydrogenated polyoxyl castor oil is done at a temperature of 55°C±2°C to form a mixture A; and mixture A is then lowered to a temperature not higher than 35°C±2°C prior to the complete dissolution of the cyclosporine. Further, the method comprises adding octoxynol-40 and then mixing the resulting mixture with an aqueous vehicle at 35°C±2°C. In another aspect, the aqueous vehicle is mixed at a temperature of 55±2°C.

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising cyclosporine, a hydrogenated polyoxyl castor oil, and octoxynol-40, wherein the ophthalmic formulation is made by a method comprising the steps of:
a) mixing the cyclosporine with a hydrogenated polyoxyl castor oil at a temperature of 55°C or above to form a mixture A; and
b) keeping mixture A under vacuum to remove foam;
c) optionally, lowering the temperature of mixture A to a temperature of 35°C±2°C prior to the complete dissolution of the cyclosporine, adding octoxynol-40; and
d) mixing the resulting mixture with the aqueous vehicle.

Preferably, mixing the cyclosporine with a hydrogenated polyoxyl castor oil is done at a temperature of 55°C-60°C to form a mixture A. Optionally, mixture A is lowered to a temperature 35°C- 40°C prior to the complete dissolution of the cyclosporine. More preferably, mixing the cyclosporine with the hydrogenated polyoxyl castor oil is done at a temperature of 55°C±2°C to form a mixture A; and optionally lowering the temperature to not higher than 35°C±2°C prior to the complete dissolution of the cyclosporine. Further, the method comprises adding octoxynol-40 and then mixing the resulting mixture with an aqueous vehicle at 35°C±2°C. In another aspect, the aqueous vehicle is mixed at a temperature of 55±2°C.

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a stable nanomicellar ophthalmic formulation comprising cyclosporine, a hydrogenated polyoxyl castor oil, and octoxynol-40, wherein the ophthalmic formulation is made by a method comprising the steps of:
a) mixing the cyclosporine with the hydrogenated polyoxyl castor oil at a temperature of 55°C or above to form a mixture A; and
b) lowering the temperature of mixture A to a temperature not higher than 40°C prior to the complete dissolution of the cyclosporine.

Preferably, mixing the cyclosporine with a hydrogenated polyoxyl castor oil is done at a temperature of 55°C-60°C to form a mixture A. Preferably, mixture A is lowered to a temperature 35°C- 40°C prior to the complete dissolution of the cyclosporine. More preferably, mixing the cyclosporine with the hydrogenated polyoxyl castor oil is done at a temperature of 55°C±2°C to form a mixture A; and then mixture A is lowered to a temperature not higher than 35°C±2°C prior to the complete dissolution of the cyclosporine. Further, the method comprises adding octoxynol-40 and then mixing the resulting mixture with an aqueous vehicle at 35°C±2°C. In another aspect, the aqueous vehicle is mixed at a temperature of 55±2°C.

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure relates to a method of making a stable nanomicellar ophthalmic formulation comprising:
cyclosporine, a polyoxyl lipid or fatty acid, and a polyalkoxylated alcohol, wherein the ophthalmic formulation is made by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine;
c) adding octoxynol-40; and
d) mixing the resulting mixture with the aqueous vehicle.

Another embodiment of the present disclosure is a stable nanomicellar ophthalmic formulation comprising cyclosporine, a hydrogenated polyoxyl castor oil, and octoxynol-40, wherein the ophthalmic formulation is made by a method comprising the steps of:
a) mixing the cyclosporine with a hydrogenated polyoxyl castor oil at a temperature of 55°C or above to form a mixture A; and
b) keeping mixture A under vacuum to remove foam;
c) optionally, lowering the temperature of mixture A to a temperature of 35°C±2°C prior to the complete dissolution of the cyclosporine;
d) adding octoxynol-40; and
e) mixing the resulting mixture with the aqueous vehicle.

Preferably, mixing the cyclosporine with a hydrogenated polyoxyl castor oil is done at a temperature of 55°C-60°C to form a mixture A. Optionally, mixture A is lowered to a temperature 35°C- 40°C prior to the complete dissolution of the cyclosporine. More preferably, mixing the cyclosporine with the hydrogenated polyoxyl castor oil is done at a temperature of 55°C±2°C to form a mixture A; and optionally lowering the temperature to not higher than 35°C±2°C prior to the complete dissolution of the cyclosporine. Further, the method comprises adding octoxynol-40 and then mixing the resulting mixture with an aqueous vehicle at 35°C±2°C. In another aspect, the aqueous vehicle is mixed at a temperature of 55±2°C.

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a stable nanomicellar ophthalmic formulation comprising: cyclosporine, hydrogenated 40 polyoxyl castor oil, octoxynol-40, and an aqueous vehicle,
wherein the ophthalmic formulation is made by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine;
c) adding octoxynol-40; and
d) mixing the resulting mixture with the aqueous vehicle.

In one aspect, mixing the aqueous vehicle to a temperature of at 35°C±2°C. In another aspect, mixing the aqueous vehicle to a temperature of 55±2°C.

In one embodiment, the present invention provides a stable nanomicellar ophthalmic formulation comprising cyclosporine form with characteristic XRD peaks at 2-theta (deg.) 6.9, 7.8, 9.4 and 15.9.

In another embodiment, the present invention provides a stable nanomicellar ophthalmic formulation comprising an amorphous form of cyclosporine.

Another embodiment of the present disclosure is a method of making a stable nanomicellar ophthalmic formulation comprising a cyclosporine, a hydrogenated polyoxyl castor oil, and octoxynol-40, wherein the ophthalmic formulation is made by a method comprising the steps of: mixing the cyclosporine with a hydrogenated polyoxyl castor oil at a temperature of 55°C or above to form a mixture A; and preventing the formation of the cyclosporine form with characteristic XRD peaks at 2-theta (deg.) 7.4, 8.7, 14.4 and 17.5 (B in Figure 2) by altering the temperature of mixture A prior to the complete dissolution of the cyclosporine.

Preferably, mixing the cyclosporine with a hydrogenated polyoxyl castor oil is done at a temperature of 55°C-60°C to form a mixture A. Further, mixture A is lowered to a temperature of 35°C- 40°C prior to the complete dissolution of the cyclosporine. More preferably, mixing the cyclosporine with the hydrogenated polyoxyl castor oil is done at a temperature of 55°C±2°C to form a mixture A; and then mixture A is lowered to a temperature not higher than 35°C±2°C prior to the complete dissolution of the cyclosporine. In one aspect, the stable nanomicellar ophthalmic formulation comprises cyclosporine with characteristic XRD peaks at 2-theta (deg.) 6.9, 7.8, 9.4 and 15.9 (A in Figure 2). In another aspect, the stable nanomicellar ophthalmic formulation comprises an amorphous form of cyclosporine. Further, the method comprises adding the octoxynol-40 and then mixing the resulting mixture with an aqueous vehicle at 35°C±2°C. In another aspect, the resulting mixture is mixed with the aqueous vehicle at a temperature of 55±2°C. Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising a cyclosporine, a hydrogenated polyoxyl castor oil, and octoxynol-40, wherein the ophthalmic formulation is made by a method comprising the steps of: mixing the cyclosporine with the hydrogenated polyoxyl castor oil at a temperature of 55°C or above to form a mixture A; and preventing the formation of the cyclosporine form with characteristic XRD peaks at 2-theta (deg.) 8.5, 9.3, 11.6 and 20.3 (C in Figure 2) by altering the temperature of mixture A prior to the complete dissolution of the cyclosporine.

Preferably, mixing the cyclosporine with a hydrogenated polyoxyl castor oil is done at a temperature of 55°C-60°C to form a mixture A. Further, mixture A is then lowered to a temperature of 35°C- 40°C prior to the complete dissolution of the cyclosporine. More preferably, mixing the cyclosporine with the hydrogenated polyoxyl castor oil is done at a temperature of 55°C±2°C to form a mixture A; and then the temperature of mixture A is lowered to a temperature not higher than 35°C±2°C prior to the complete dissolution of the cyclosporine In one aspect, the method of making a stable nanomicellar ophthalmic formulation comprises cyclosporine with characteristic XRD peaks at 2-theta (deg.) 6.9, 7.8, 9.4 and 15.9 (A in Figure 2). In another aspect, the method of making a stable nanomicellar ophthalmic formulation comprises an amorphous form of cyclosporine. Further, the method comprises adding octoxynol-40 and then mixing the resulting mixture with an aqueous vehicle at 35°C±2°C. In another aspect, the aqueous vehicle is mixed at a temperature of 55±2°C. Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising cyclosporine, a hydrogenated polyoxyl castor oil, and octoxynol-40, wherein the ophthalmic formulation is made by a method comprising the steps of: mixing the cyclosporine with hydrogenated polyoxyl castor oil at a temperature of 55°C or above to form a mixture A; and applying vacuum to the mixture to prevent the formation of the cyclosporine form with characteristic XRD peaks at 2-theta (deg.) 7.4, 8.7, 14.4 and 17.5 (B in Figure 2).

Preferably, mixing the cyclosporine with hydrogenated polyoxyl castor oil is done at a temperature of 55°C-60°C to form a mixture A. Optionally, the mixture A is lowered to a temperature 35°C- 40°C prior to the complete dissolution of the cyclosporine. More preferably, mixing the cyclosporine with the hydrogenated polyoxyl castor oil is done at a temperature of 55°C±2°C to form a mixture A; optionally lowering to a temperature not higher than 35°C±2°C prior to the complete dissolution of the cyclosporine. Further, the method comprises adding the octoxynol-40 and then mixing the resulting mixture with an aqueous vehicle at 35°C±2°C. In another aspect, the resulting mixture is mixed with the aqueous vehicle at a temperature of 55±2°C. Preferably, cyclosporine is present in cyclosporine form with characteristic XRD peaks at 2-theta (deg.) 6.9, 7.8, 9.4 and 15.9 (A in Figure 2). In another embodiment, preferably, cyclosporine is present in amorphous form. Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising cyclosporine, a hydrogenated polyoxyl castor oil, and octoxynol-40, wherein the ophthalmic formulation is made by a method comprising the steps of: mixing the cyclosporine with hydrogenated polyoxyl castor oil at a temperature of 55°C or above to form a mixture A; and applying vacuum to the mixture to prevent the formation of the cyclosporine form with characteristic XRD peaks at 2-theta (deg.) 8.5, 9.3, 11.6 and 20.3 (C in Figure 2).

Preferably, mixing the cyclosporine with hydrogenated polyoxyl castor oil is done at a temperature of 55°C-60°C to form a mixture A. Optionally, the mixture A is lowered to a temperature 35°C- 40°C prior to the complete dissolution of the cyclosporine. More preferably, mixing the cyclosporine with hydrogenated polyoxyl castor oil is done at a temperature of 55°C±2°C to form a mixture A; optionally lowering to a temperature not higher than 35°C±2°C prior to the complete dissolution of the cyclosporine. Further, the method comprises adding the octoxynol-40 and then mixing the resulting mixture with an aqueous vehicle at 35°C±2°C. In another aspect, the resulting mixture is mixed with the aqueous vehicle at a temperature of 55±2°C. Preferably, cyclosporine is present in cyclosporine form with characteristic XRD peaks at 2-theta (deg.) 6.9, 7.8, 9.4 and 15.9 (A in Figure 2). In another embodiment, preferably, cyclosporine is present in amorphous form. Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising:
cyclosporine, hydrogenated polyoxyl castor oil, octoxynol-40, and an aqueous vehicle, wherein the method comprising the steps of:
a) mixing the cyclosporine with the hydrogenated polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of at 127-130°C;
wherein, cyclosporine is present in a form having characteristic XRD peaks at 2-theta (deg.) 7.4, 8.7, 14.4 and 17.5 (B in Figure 2).

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a method of making a stable nanomicellar ophthalmic formulation comprising:
cyclosporine, hydrogenated polyoxyl castor oil, octoxynol-40, and an aqueous vehicle, wherein the method comprising the steps of:
a) mixing the cyclosporine with the hydrogenated polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of 127-130°C,
wherein, cyclosporine is present in a form with characteristic XRD peaks at 2-theta (deg.) 8.5, 9.3, 11.6 and 20.3 (C in Figure 2). Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

In yet another embodiment of the present disclosure is a stable nanomicellar ophthalmic formulation comprising:
cyclosporine, hydrogenated polyoxyl castor oil, octoxynol-40, and an aqueous vehicle, wherein the ophthalmic formulation is made by a method comprising the steps of:
a) mixing the cyclosporine with the hydrogenated polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of at 127-130°C;
wherein, cyclosporine is present in a form having characteristic XRD peaks at 2-theta (deg.) 7.4, 8.7, 14.4 and 17.5 (B in Figure 2).

Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

In yet another embodiment of the present disclosure is a stable nanomicellar ophthalmic formulation comprising:
cyclosporine, hydrogenated polyoxyl castor oil, octoxynol-40, and an aqueous vehicle, wherein the ophthalmic formulation is made by a method comprising the steps of:
a) mixing the cyclosporine with the hydrogenated polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of 127-130°C,
wherein, cyclosporine is present in a form with characteristic XRD peaks at 2-theta (deg.) 8.5, 9.3, 11.6 and 20.3 (C in Figure 2). Preferably, the hydrogenated polyoxyl castor oil is hydrogenated 40 polyoxyl castor oil (HCO-40). More preferably, the ophthalmic formulation comprises 0.09 wt % of cyclosporine, 1.0 wt % of hydrogenated 40 polyoxyl castor oil, and 0.05 wt % of octoxynol-40.

Another embodiment of the present disclosure is a stable nanomicellar ophthalmic formulation comprising cyclosporine, a hydrogenated 40 polyoxyl castor oil (HCO-40), and octoxynol-40, wherein the formulation is a solution; and the formulation exhibits stability at room temperature (20-25°C) for 6 to at least 24 months. Typically, the ophthalmic formulations are stable when maintained at room temperature for at least 6 months, at least 8 months, at least 10 months, at least 12 months, at least 14 months, at least 16 months, at least 18 months, at least 20 months and at least 24 months. More preferably, the stable nanomicellar ophthalmic formulation is a solution and comprises 0.09 wt % of cyclosporine, 1.0 wt % of hydrogenated 40 polyoxyl castor oil, and 0.05 wt % of octoxynol-40.

The cyclosporine present in certain formulations according to embodiments of this disclosure is preferably amorphous when it is in solution. Alternatively, the cyclosporine may be present as a cyclosporine form with characteristic XRD peaks at 2-theta (deg.) 6.9, 7.8, 9.4 and 15.9 in solution. In certain embodiments, there is no crystalline cyclosporine present in a solution according to the present disclosure. In certain embodiment, the formulations according to embodiments of this disclosure is substantially free of a cyclosporine form with characteristic XRD peaks at 2-theta (deg.) 7.4, 8.7, 14.4 and 17.5. In alternative embodiments, the formulations according to this disclosure are substantially free of a cyclosporine form with characteristic XRD peaks at 2-theta (deg.) 8.5, 9.3, 11.6 and 20.3.

### Additional Formulation Ingredients

The compositions of the present disclosure may also contain other components such as, but not limited to, additives, adjuvants, buffers, tonicity agents, bioadhesive polymers, and preservatives. In any of the compositions of this disclosure for topical to the eye, the mixtures are preferably formulated at about pH 5 to about pH 8. This pH range may be achieved by the addition of buffers to the composition as described in the examples. In an embodiment, the pH range in the composition in a formulation is about pH 6.5 to about pH 7.2. It should be appreciated that the compositions of the present disclosure may be buffered by any common buffer system such as phosphate, borate, acetate, citrate, carbonate and borate-polyol complexes, with the pH and osmolality adjusted in accordance with well-known techniques to proper physiological values. The mixed micellar compositions of the present disclosure are stable in buffered aqueous solution. That is, there is no adverse interaction between the buffer and any other component that would cause the compositions to be unstable.

Tonicity agents include, for example, mannitol, sodium chloride, sodium nitrate, sodium sulfate, dextrose, xylitol or combinations thereof. These tonicity agents may be used to adjust the osmolality of the compositions. In an aspect, the osmolality of the formulation is adjusted to be in the range of about 150 to about 200 mOsmol/kg. In a preferred aspect, the osmolality of the formulation is adjusted to between about 160 to about 190 mOsmol/kg.

An additive such as a sugar, a glycerol, and other sugar alcohols, can be included in the compositions of the present disclosure. Pharmaceutical additives can be added to increase the efficacy or potency of other ingredients in the composition. For example, a pharmaceutical additive can be added to a composition of the present disclosure to improve the stability of the calcineurin inhibitor, to adjust the osmolality of the composition, to adjust the viscosity of the composition, or for another reason, such as effecting drug delivery. Non-limiting examples of pharmaceutical additives of the present disclosure include sugars, such as, trehalose, mannose, D-galactose, and lactose. In an embodiment, the sugars can be incorporated into a composition prior to hydrating the thin film (i.e. internally). In another embodiment, the sugars can be incorporated into a composition during the hydration step (i.e. externally). In an embodiment, an aqueous, clear, mixed micellar solution of the present disclosure includes additives such as sugars.

In an embodiment, compositions of the present disclosure further comprise one or more bioadhesive polymers. Bioadhesion refers to the ability of certain synthetic and biological macromolecules and hydrocolloids to adhere to biological tissues. Bioadhesion is a complex phenomenon, depending in part upon the properties of polymers, biological tissue, and the surrounding environment. Several factors have been found to contribute to a polymer's bioadhesive capacity: the presence of functional groups able to form hydrogen bridges (--OH, COOH), the presence and strength of anionic charges, sufficient elasticity for the polymeric chains to interpenetrate the mucous layer, and high molecular weight. Bioadhesion systems have been used in dentistry, orthopedics, ophthalmology, and in surgical applications. However, there has recently emerged significant interest in the use of bioadhesive materials in other areas such as soft tissue-based artificial replacements, and controlled release systems for local release of bioactive agents. Such applications include systems for release of drugs in the buccal or nasal cavity, and for intestinal or rectal administration.

In an embodiment, a composition of the present disclosure includes at least one bioadhesive polymer. The bioadhesive polymer can enhance the viscosity of the composition and thereby increase residence time in the eye. Bioadhesive polymers of the present disclosure include, for example, carboxylic polymers like Carbopol^{®} (carbomers), Noveon^{®} (polycarbophils), cellulose derivatives including alkyl and hydroxyalkyl cellulose like methylcellulose, hydroxypropylcellulose, carboxymethylcellulose, gums like locust beam, xanthan, agarose, karaya, guar, and other polymers including but not limited to polyvinyl alcohol, povidone, polyethylene glycol, Pluronic^{®} (Poloxamers), tragacanth, and hyaluronic acid; phase-transition polymers for providing sustained and controlled delivery of enclosed medicaments to the eye (e.g., alginic acid, carrageenans (e.g., Eucheuma), xanthan and locust bean gum mixtures, pectins, cellulose acetate phthalate, alkylhydroxyalkyl cellulose and derivatives thereof, hydroxyalkylated polyacrylic acids and derivatives thereof, poloxamers and their derivatives, etc. Physical characteristics in these polymers can be mediated by changes in environmental factors such as ionic strength, pH, or temperature alone or in combination with other factors. In an embodiment, the optional one or more bioadhesive polymers is present in the composition from about 0.01 wt % to about 10 wt %/volume, preferably from about 0.1 to about 5 wt %/volume. In an embodiment, the compositions of the present disclosure further comprise at least one hydrophilic polymer excipient selected from, for example, PVP-K-30, PVP-K-90, HPMC, HEC, and polycarbophil. In an embodiment, the polymer excipient is selected from PVP-K-90, PVP-K-30 or HPMC. In an embodiment, the polymer excipient is selected from PVP-K-90 or PVP-K-30.

In an embodiment, if a preservative is desired, the compositions may optionally be preserved with any of many well-known preservatives, including benzyl alcohol with/without EDTA, benzalkonium chloride, chlorhexidine, Cosmocil^{®} CQ, or Dowicil^{®} 200. In certain embodiments, it may be desirable for a formulation as described herein to not include any preservatives. In this regard, preservatives may in some embodiments not be necessary or desirable in formulations included in single use containers. In other embodiments, it may be advantageous to include preservatives, such as in certain embodiments in which the formulations are included in a multiuse container.

In a preferable embodiment, the present disclosure relates to a method of making a stable nanomicellar ophthalmic formulation comprising:
0.09 wt % of cyclosporine
about 1.0 wt % of hydrogenated 40 polyoxyl castor oil,
about 0.05 wt % of octoxynol-40, and
an aqueous vehicle,
wherein the ophthalmic formulation is made by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40; and
c) mixing the resulting mixture with the aqueous vehicle.

In another preferable embodiment, the present disclosure relates to a method of making a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40; and
e) mixing the resulting mixture with the aqueous vehicle.

In one aspect, mixing the aqueous vehicle at a temperature of at 35°C±2°C. In another aspect, mixing the aqueous vehicle at a temperature of 55±2°C.

The stable nanomicellar ophthalmic formulation of preceding embodiments further comprises:
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection.

In another preferable embodiment, the present disclosure is a stable nanomicellar ophthalmic formulation, wherein the pH of the formulation is about 5.0 to 8.0. More preferably, the pH of the formulation is about 6.5 to 7.2.

Further, the present invention disclosure is a stable nanomicellar ophthalmic formulation wherein the osmolality of the formulation is between about 150 to about 200 mOsmol/kg.

Further, the present invention disclosure is a stable nanomicellar ophthalmic formulation wherein the mixed nanomicellar size and polydispersity index are determined with Zetasizer, Malvern Instruments, N.J. In brief, approximately 1 ml of each formulation was transferred to a cuvette and placed in the instrument. A laser beam of light was used to determine the mixed nanomicellar size. Nanomicelles contemplated by the present disclosure typically have a particle size in the range of about 1-100 nm; in some embodiments, the particle size falls in the range of about 5-50 nm; in some embodiments, the particle size falls in the range of about 10-40 nm; in some embodiments, the particle size is about 13-16 nm.

In another embodiment, the present disclosure relates to a cyclosporine form with characteristic XRD peaks at 2-theta (deg.) 6.9, 7.8, 9.4 and 15.9.

In yet another embodiment, the present disclosure relates to a stable nanomicellar ophthalmic formulation comprising an amorphous form of cyclosporine.

In yet another embodiment, the present disclosure relates to a stable nanomicellar ophthalmic formulation, wherein the formulation is substantially free of a cyclosporine form with characteristic XRD peaks at 2-theta (deg.) 7.4, 8.7, 14.4 and 17.5.

In yet another embodiment, the present disclosure is a stable nanomicellar ophthalmic formulation, wherein the formulation is substantially free of a cyclosporine form with characteristic XRD peaks at 2-theta (deg.) 8.5, 9.3, 11.6 and 20.3.The XRD data for these cyclosporine forms are presented in Figure 2.

The compete dissolution time of cyclosporine depends on quantity of the cyclosporine A to be dissolved based on the batch size. Generally dissolution time of cyclosporine in hydrogenated 40 polyoxyl castor oil (KOLLIPHOR RH 40) at a ratio of 9:10 (cyclosporine : KOLLIPHOR RH 40) was found to be not less than 130 minutes. The cyclosporine A slowly dissolved over a period of time with stirring and during this complete dissolution period, the solution became clear. If the cyclosporine A re-precipitates during the dissolution period, there is a chance that a slight turbid solution might have been transferred to the aqueous phase during manufacturing of the batch. This may initiate a seeding effect for crystal growth in the final formulation during storage, which may lead to batch failure. Based on these observations, the solubility behavior of cyclosporine was studied in hydrogenated 40 polyoxyl castor oil (Kolliphor RH 40) at 55°C and 35°C. It was found that the solution stability at 35°C was comparatively higher than the solution stability at 55°C (see Example 2 in Table 4). Based on the above observation it is believed that if cyclosporine was not completely dissolved in Kolliphor RH 40 at 55°C, the temperature can be reduced to 35°C. This is because the solution stability of cyclosporine at 35°C was higher than that of 55°C.

In another aspect, when the cyclosporine is present in a form having characteristic XRD peaks at 2-theta (deg.) 7.4, 8.7, 14.4 and 17.5 (B in Figure 2), the mixing of the cyclosporine with the hydrogenated polyoxyl castor oil is done at a temperature of 127-130°C until completely dissolved and octoxynol-40 is added to this to mixture at 127-130°C.

In another aspect, when the cyclosporine is present in a form having characteristic XRD peaks at 2-theta (deg.) 8.5, 9.3, 11.6 and 20.3 (C in Figure 2)., the mixing of the cyclosporine with the hydrogenated polyoxyl castor oil is done at a temperature of 127-130°C until completely dissolved and octoxynol-40 is added to this to mixture at 127-130°C.

In certain aspects of the present invention, in the step of lowering the temperature of mixture A, mixture A is lowered to a temperature of 35°C±2°C. The step of lowering the temperature of mixture A may occur in less than 65 minutes. Preferably, at about 60 mins. In certain aspects, water may be added after the step of lowering the temperature of mixture A.

In another aspect, the present disclosure relates to a stable nanomicellar ophthalmic formulation, prepared by a method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine;
c) adding octoxynol-40; and
d) then mixing the resulting mixture with an aqueous vehicle at 35°C±2°C, wherein the mixture A is lowered to a temperature of 35°C±2°C in less than 65 minutes. Preferably, the mixture A is lowered to a temperature of 35°C±2°C in 60 minutes.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of to 35°C±2°C and stirring for 60-70 minutes. Preferably, stirring the mixture for 60±5 minutes at a temperature of 35°C±2°C.

In yet another aspect, the present invention relates to a method of making a stable nanomicellar ophthalmic formulation comprising:
0.09 wt % cyclosporine,
about 1.0 wt % hydrogenated 40 polyoxyl castor oil,
about 0.05 wt % octoxynol-40,
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection,
wherein the method comprising the steps of:
a) mixing the cyclosporine with the hydrogenated 40 polyoxyl castor oil at a temperature of 55°C±2°C or above to form a mixture A;
b) lowering the temperature of mixture A to a temperature of 35°C±2°C prior to the complete dissolution of the cyclosporine, adding octoxynol-40 to form an API mixture;
c) adding API mixture to Water for Injection (WFI);
d) adding remaining excipients to step (c) in the order of sodium phosphate monobasic, then sodium phosphate dibasic, then sodium chloride, and then polyvinylpyrrolidone;
e) adjusting the pH to 6.5 to 7.2 and bringing to the final volume with WFI.

In one aspect, mixing cyclosporine in step (a) at 200-300 RPM.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of the mixture A to 35°C±2°C in less than 65 minutes. Preferably, lowering the temperature of the mixture A to 35°C±2°C in 40-50 minutes.

In another aspect, stirring the mixture of step (b) for 60-70 minutes. Preferably, stirring the mixture for 60±5 minutes at a temperature of 35°C±2°C.

In one aspect, mixing the API mixture to WFI at a temperature of 35°C±2°C. In another aspect, mixing the API mixture to WFI at a temperature of 55±2°C.

In yet another aspect, the present invention relates to a method of making a stable nanomicellar ophthalmic formulation comprising:
0.09 wt % cyclosporine,
about 1.0 wt % hydrogenated 40 polyoxyl castor oil,
about 0.05 wt % octoxynol-40,
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid to adjust the pH, and
water for injection,
wherein the method comprising the steps of:
a) mixing the cyclosporine with the hydrogenated 40 polyoxyl castor oil at a temperature of 55°C±2°C or above to form a mixture A;
b) keeping mixture A under vacuum to remove foam;
c) optionally, lowering the temperature of mixture A to a temperature of 35°C±2°C prior to the complete dissolution of the cyclosporine;
d) adding octoxynol-40;
e) adding API mixture to Water for Injection (WFI);
f) adding remaining excipients to step (c) in the order of sodium phosphate monobasic, then sodium phosphate dibasic, then sodium chloride, and then polyvinylpyrrolidone; and
g) adjusting the pH to 6.5 to 7.2 and bringing to the final volume with WFI.

In one aspect, mixing the mixture A of step (a) for 20-30 minutes. Preferably, 20-25 minutes, more preferably, mixing the mixture A of step (a) for 20 ±2 minutes.

In one aspect, lowering the temperature of the mixture A to 35°C±2°C in less than 65 minutes. Preferably, lowering the temperature of the mixture A to 35°C±2°C in 40-50 minutes.

In one aspect, mixing the API mixture to WFI at a temperature of 35°C±2°C. In another aspect, mixing the API mixture to WFI at a temperature of 55±2°C.

In a more preferred aspect, the present invention provides for a stable nanomicellar ophthalmic formulation prepared by any of the method as described above.

In yet another aspect, the present disclosure relates to a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of at 127-130°C,
wherein, cyclosporine is present in a form having characteristic XRD peaks at 2-theta (deg.) 7.4, 8.7, 14.4 and 17.5.

In yet another aspect, the present disclosure relates to a stable nanomicellar ophthalmic formulation comprising:
0.09 wt % cyclosporine,
about 1.0 wt % hydrogenated 40 polyoxyl castor oil,
about 0.05 wt % octoxynol-40, and
an aqueous vehicle,
wherein the ophthalmic formulation is made by a method comprising the steps of:
a) mixing the cyclosporine with the hydrogenated 40 polyoxyl castor oil at a temperature of 127-130°C until completely dissolved to form a mixture A;
b) adding octoxynol-40 to mixture A at 127-130°C; and
c) mixing the resulting mixture with the aqueous vehicle at a temperature of 127-130°C,
wherein, cyclosporine is present in a form with characteristic XRD peaks at 2-theta (deg.) 8.5, 9.3, 11.6 and 20.3.

In one embodiment, the mixing speed, time and energy input plays a role in the complete dissolution of cyclosporine in hydrogenated polyoxyl castor oil. When low speed is used, time is increased for dissolution, In one aspect of the embodiment, typically cyclosporine is dissolved hydrogenated polyoxyl castor oil by stirring at approximately 200-300 RPM for 75 minutes, for 70 minutes, for 65minutes, for 60 minutes, for 55 minutes, for 50 minutes, for 45 minutes, for 40 minutes, for 35 minutes, for 30 minutes, for 25 minutes, for 20 minutes, for 15 minutes, for 10 minutes. In another aspect, typically cyclosporine is dissolved hydrogenated polyoxyl castor oil by stirring at approximately 300-400 RPM for 65minutes, for 60 minutes, for 55 minutes, for 50 minutes, for 45 minutes, for 40 minutes, for 35 minutes, for 30 minutes, for 25 minutes, for 20 minutes, for 15 minutes, for 10 minutes. In another aspect, typically cyclosporine is dissolved hydrogenated polyoxyl castor oil by stirring at approximately 350-400 RPM for 60 minutes, for 55 minutes, for 50 minutes, for 45 minutes, for 40 minutes, for 35 minutes, for 30 minutes, for 25 minutes, for 20 minutes, for 15 minutes, for 10 minutes. In another aspect, typically cyclosporine is dissolved hydrogenated polyoxyl castor oil by stirring at approximately 400-450 RPM for 50 minutes, for 45 minutes, for 40 minutes, for 35 minutes, for 30 minutes, for 25 minutes, for 20 minutes, for 15 minutes, for 10 minutes. In another aspect, typically cyclosporine is dissolved hydrogenated polyoxyl castor oil by stirring at approximately >450 RPM for 40 minutes, for 35 minutes, for 30 minutes, for 25 minutes, for 20 minutes, for 15 minutes, for 10 minutes, for 5 minutes. In yet another aspect, cyclosporine is dissolved hydrogenated polyoxyl castor oil by stirring at approximately 200-300 RPM till complete dissolution.

The energy input into the mixture is defined in Equation 1 as: $E / V = {n}^{3} {D}^{5} t / V$ (1) wherein E is the theoretical energy input, n is the shear plate rpm, D is the shear plate diameter, t is the time, and V is the solution volume. The energy input per volume is scale independent. (Diaz, M., et al., "Mixing Power, External Convection, and Effectiveness in Bioreactors," Biotechnology and Bioengineering, Vol. 51, 1996, pp. 131-140). This is a simple, rapid and reliable way to scale up the preparation of the nanomicellar formulation.

Alternative methods of mixing, such as the use of sonicators, or the use of solvents, water, or pressure, may be used to impact the temperatures or times of the respective method steps. For example, if solvents are used, it may be possible to utilize lower temperatures in the method steps.

The instant disclosure further relates to treating or preventing ocular diseases or disorders, for example, by local administration of the formulations as described herein.

Any references to methods of treatment by therapy or *in vivo* diagnosis methods of this description is to be interpreted as a reference to compounds, pharmaceutical compositions and medicaments of the present invention for use in those methods.

The term "treating" refers to: preventing a disease, disorder or condition from occurring in a cell, a tissue, a system, animal or human which may be predisposed to the disease, disorder and/or condition but has not yet been diagnosed as having it; stabilizing a disease, disorder or condition, i.e., arresting its development; and/or relieving one or more symptoms of the disease, disorder or condition, i.e., causing regression of the disease, disorder and/or condition.

As used herein, a composition that "prevents" a disorder or condition refers to a compound that, in a statistical sample, reduces the occurrence of the disorder or condition in the treated sample relative to an untreated control sample, or delays the onset or reduces the severity of one or more symptoms of the disorder or condition relative to the untreated control sample.

As used herein, the term "ocular disease" refers to diseases/conditions of the eye(s) that can be sight threatening, lead to eye discomfort, and may signal systemic health problems.

A patient or subject to be treated by any of the compositions or methods of the present disclosure can mean either a human or a non-human animal. In an embodiment, the present disclosure provides methods for the treatment of an ocular disease in a human patient in need thereof. In an embodiment, the present disclosure provides methods for the treatment of an inflammatory ocular disease in a human patient in need thereof. In another embodiment, the present disclosure provides methods for the treatment of an ocular disease in a veterinary patient in need thereof, including, but not limited to dogs, horses, cats, rabbits, gerbils, hamsters, rodents, birds, aquatic mammals, cattle, pigs, camelids, and other zoological animals.

In some embodiments of the compositions and methods disclosed herein, the cyclosporine further comprises one or more additional active ingredients, e.g., active agents selected from the group consisting of a resolvin or resolvin-like compound, a steroid (such as a corticosteroid), and the like. In some embodiments, the additional active agent includes a resolvin. In some embodiments, the additional active agent includes a corticosteroid. In some embodiments, the additional active agent includes a resolvin and a corticosteroid. In some embodiments, the additional active agent includes an antibiotic, for example one or more antibiotics selected from the group consisting of azythromycin, ciprofloxacin, ofloxacin, gatifloxacin, levofloxacin, moxifloxacin, besifloxacin, and levofloxacin. In some embodiments, the additional active agent includes an antibiotic, for example one or more antibiotics selected from the group consisting of azythromycin, ciprofloxacin, ofloxacin, gatifloxacin, levofloxacin, moxifloxacin, besifloxacin, and levofloxacin; and a second of such agents is a resolvin such as described herein (including without limitation compound 1001). In some embodiments, the active agent includes two or more active agents and one of said active agents is an antiviral, for example one or more antivirals selected from the group consisting of ganciclovir, trifluridine, acyclovir, famciclovir, valacyclovir, penciclovir and cidofovir. In some embodiments, the active agent includes two or more active agents and one of the active agents is an antibiotic, for example one or more antivirals selected from the group consisting of ganciclovir, trifluridine, acyclovir, famciclovir, valacyclovir, penciclovir and cidofovir; and a second of the active agents is a resolvin such as described herein (including without limitation compound 1001).

Accordingly, in another aspect, provided is a method treating or preventing an ocular disease or condition that includes locally administering a formulation of any of the aspects or embodiments as disclosed herein. In some embodiments, the ocular disease is an anterior segment disease. In some embodiments, the ocular disease is a posterior segment disease. In some embodiments, the ocular disease is one or more selected from the group consisting of dry eye syndrome, Sjogren's syndrome, uveitis, anterior uveitis (iritis), chorioretinitis, posterior uveitis, conjunctivitis, allergic conjunctivitis, keratitis, keratoconjunctivitis, vernal keratoconjunctivitis (VKC), atopic keratoconjunctivitis, systemic immune mediated diseases such as cicatrizing conjunctivitis and other autoimmune disorders of the ocular surface, blepharitis, scleritis, age-related macular degeneration (AMD), diabetic retinopathy (DR), diabetic macular edema (DME), ocular neovascularization, age-related macular degeneration (ARMD), proliferative vitreoretinopathy (PVR), cytomegalovirus (CMV) retinitis, optic neuritis, retrobulbar neuritis, and macular pucker. In one embodiment, the ocular disease is dry eye. In one embodiment, the ocular disease is allergic conjunctivitis. In one embodiment. the ocular disease is age-related macular degeneration (AMD). In one embodiment, the ocular disease is diabetic retinopathy.

The daily dose of the ophthalmic formulation, effective to reduce dry eye symptoms and/or to improve tear film can be divided among one or several unit dose administrations. A subject would use the product as needed, but generally, this would not be more than twice a day and in many instances the product would be used only once a day. A preferred regimen for the nanomicellar ophthalmic formulation of the present invention is one drop of 0.09% (w/w) solution per eye twice a day (approximately 12 hours apart).

### EXAMPLES

To illustrate non-limiting embodiments of the present disclosure, the following Examples were prepared.

### EXAMPLE 1

**Table 1: Cyclosporine nanomicellar ophthalmic formulation**

| **Ingredients** | **Amount** |
|---|---|
| Cyclosporine | 0.09 wt % |
| hydrogenated 40 polyoxyl castor oil, | 1.0 wt % |
| octoxynol-40 | 0.05 wt % |
| sodium phosphate monobasic, | 0.53 wt % |
| sodium phosphate dibasic, | 0.47 wt % |
| sodium chloride, | 0.05 wt % |
| povidone, | 0.3 wt % |
| sodium hydroxide | q.s. to adjust pH, if required |
| hydrochloric acid | q.s. to adjust pH, if required |
| water for injection | q.s. to 100% |

Cyclosporine nanomicellar ophthalmic solutions were prepared as follows. In Example 1(a), polyoxyl 40 hydrogenated castor oil (KOLLIPHOR RH 40) was melted at 55-60°C with stirring at around 200 rpm. Cyclosporine A was added to the melted Kolliphor RH 40 at 55-60°C and the reaction mixture was mixed at the same temperature range until complete dissolution. Following solubilization of the cyclosporine A, a surfactant (octoxynol-40) was added under stirring and after 10 minutes of stirring, this non-aqueous solution was delivered at 55-60°C to 90% water for injection. The temperature of the water for injection was maintained at < 22°C. Sodium phosphate monobasic, sodium phosphate dibasic, sodium chloride and povidone, were added to the bulk solution sequentially under stirring until complete dissolution. Once all ingredients were completely solubilized in the bulk solution, the volume was made up to 100% with water for injection to 1 L. In Example 1(b), the procedures of Example 1(a) were followed, with the exception that the cyclosporine A solution in KOLLIPHOR RH 40 at 55-60°C was added to water just after it started showing turbidity.

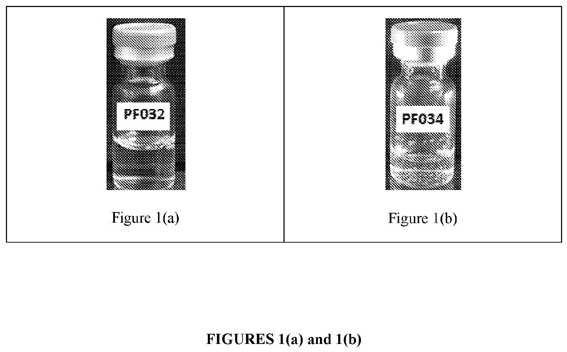

The batches of both Examples 1(a) and 1(b) were filled into 3 piece 5 mL low density polyethlylene ("LDPE") vials in an aseptic area. The vials were exposed to accelerated temperatures of 40 °C and 30 °C in chambers (to accelerate particulate formation). The samples were visually observed daily to see any sign of turbidity and/or visible particle formation. The two batches were analyzed for critical quality parameters, such as, an assay of cyclosporine, pH, osmolality, and micelle size, for which all was found well within specification. The results are given in Table 2 below. Figure 1 shows photographs of the stable batch (Example 1a) and the unstable batch (Example 1b).

**Table 2**

| **Stability Condition** | **Example 1 (a)** | **Example 1 (b)** |
|---|---|---|
| **30 °C** | **Particle/turbidity formation at 25 days** | **Particle/turbidity formation at 11 days** |
| Critical Quality Attributes | | |
| Assay of Cyclosporine | 100.05 | 100.10 |
| Total impurities | 0.470 | 0.649 |
| Ph | 6.86 | 6.79 |
| Osmolality | 167 | 171 |
| Particle Size Z avg | 16.29 | 16.95 |
| PDI | 0.148 | 0.172 |

The data in Table 2 illustrates that that when the batch is manufactured using a clear CsA non-aqueous phase (cyclosporine in KOLLIPHOR RH 40 and octoxynol-40) and then adding the mixture to a water phase, the batch remains stable for a longer time. However, when the batch is manufactured using a turbid cyclosporine non-aqueous phase and then adding the mixture to the water phase, the batch shows lower stability. This demonstrates that a heterogeneous distribution of cyclosporine within micelles might facilitate nucleation and particle formation in a finished product upon storage.

### EXAMPLE 2

The solution stability of cyclosporine A lots in polyoxyl 40 hydrogenated castor oil (KOLLIPHOR RH 40) at 55-60°C is an important process parameter for a stable formulation. It was also surprisingly found that the solubility behavior varied over the time of the storage, as shown in Table 3.

**Table 3: Solution stability of cyclosporine in KOLLIPHOR RH 40 at 55°C**

| **Parameter** | **Solution stability of CsA in Kolliphor RH 40 at 55°C** | | | | | |
|---|---|---|---|---|---|---|
| | **Initial data at receipt of the API** | **Current data** | | | | |
| | **Study** | **Study 1** | **Study 2** | **Study 3** | **Study 4** | **Study 5** |
| Time to dissolve at 55°C (min) | 20-30 | 45 | 40 | 165# | 63 | 62 |
| Total time for which the solution remains clear at 55°C (min) after API is totally dissolved (acceptance criteria is NLT 75 min) | 81* | 60 | 90 | 7 | 99 | 102 |
| | | 52* | | | | |

| | | | | | | |
|---|---|---|---|---|---|---|
| * Average of three studies results. # Some undissolved particles remained for a long time and solution started to become slightly hazy very fast after complete solubility. | | | | | | |

### EXAMPLE 3

Similarly, the solubility behavior study of cyclosporine A in polyoxyl 40 hydrogenated castor oil (KOLLIPHOR RH 40) was tested at 35°C. Table 4 shows the results of a solubility behavior study of cyclosporine A in KOLLIPHOR RH 40 at 35°C. As can be seen from Table 4, it was found that the solution stability at 35°C was comparatively higher than the solution stability at 55°C.

**Table 4: Comparative solution stability of cyclosporine A in amorphous form in KOLLIPHOR RH 40 at 35°C and 55°C**

| **Lot No. of CsA** | **35°C** | | **55°C** | |
|---|---|---|---|---|
| | **Solution stability at 35°C** | **SD** | **Solution stability at 55°C** | **SD** |
| Lot 7 | 239 | 10 | 92.17 | 14.77 |
| Lot 8 | 304 | 12 | 99.33 | 4.62 |
| Lot 9 | 248 | 17 | 79.67 | 9.29 |
| Lot 10 | 125 | 0 | 49.67 | 17.79 |
| Lot 11 | 62 | 0 | 23.25 | 1.50 |
| Lot 12 | 235 | 7.64 | 74.33 | 4.93 |
| Lot 17 | 135 | 0 | 46 | 0.00 |
| Lot 18 | 114 | 0 | 44 | 0.00 |
| Lot 19 | 80 | 0 | 47 | 0.00 |

As can be seen from Table 4, if cyclosporine A was not completely dissolved in KOLLIPHOR RH 40 at 55°C, the temperature can be reduced to 35°C. Based on this data, it is believed that the solution stability of cyclosporine A at 35°C was higher than that of 55°C. The complete dissolution stage of cyclosporine A in KOLLIPHOR RH 40 was modified as follows:
(i) API was dispersed at 55°C±2°C for 20 min and stirred for another 15 minutes;
(ii) the temperature was then decreased to 35°C±2°C from 55°C±2°C in less than 55 min (about 40 min), and the solution was stirred at 35°C±2°C until complete dissolution of the API was achieved; and
(iii) octoxynol-40 was added with stirring and the non-aqueous phase was delivered to water for injection at 35°C±2°C.

The changes in this dissolution process can accommodate lot to lot variability of the solution stability of API in Kolliphor RH-40 at 55 °C and also variability during the storage as well.

### EXAMPLE 4

The solubility behavior of three cyclosporine forms as disclosed in Figure 2 were studied in polyoxyl 40 hydrogenated castor oil (KOLLIPHOR RH 40) at 55°C. The results are shown in Table 5.

**Table 5: Solubility of cyclosporine in KOLLIPHOR RH40 at 55 °C**

| **Parameters studied** | **API type** | | | |
|---|---|---|---|---|
| | CsA form with characteristic XRD peaks at 2-theta (deg.) 6.9, 7.8, 9.4 and 15.9. | CsA form with characteristic XRD peaks at 2-theta (deg.) 7.4, 8.7, 14.4 and 17.5 | CsA form with characteristic XRD peaks at 2-theta (deg.) 8.5, 9.3, 11.6 and 20.3. | Amorphous API |
| Time taken to dissolve at 55°C (min.) | 15 | 14 min with haziness | 15 min with haziness | 8 |
| Time at which haziness appears (min.) | 130 | NA | NA | 92 |

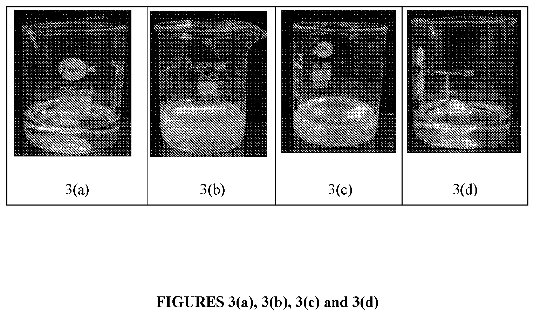

Figures 3(a) to 3(d) are photographs of the results of the study reported in Table 5.

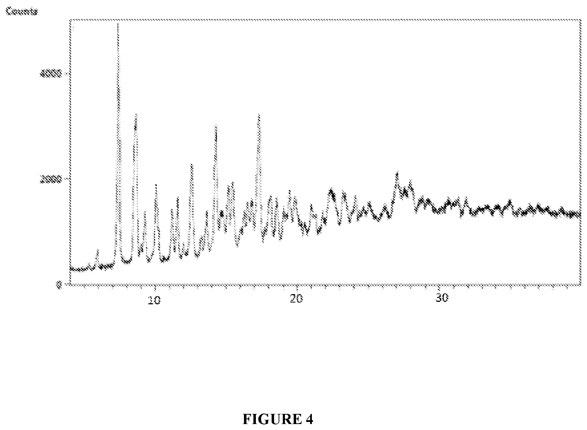

As can be seen from the Table 5 above and from the associated figures, the CsA form with characteristic XRD peaks at 2-theta (deg.) 7.4, 8.7, 14.4 and 17.5 and the CsA form with characteristic XRD peaks at 2-theta (deg.) 8.5, 9.3, 11.6 and 20.3 have much less solubility as compared to the CsA form with characteristic XRD peaks at 2-theta (deg.) 6.9, 7.8, 9.4 and 15.9. During dissolution of cyclosporine in KOLLIPHOR RH 40 at 55°C, the CsA form with characteristic XRD peaks at 2-theta (deg.) 6.9, 7.8, 9.4 and 15.9 may change into less soluble forms, or amorphous cyclosporine may recrystallize into comparatively less soluble forms. This conversion may be dependent on stresses to the system, including temperature, long storage times at higher temperatures, and the like. To this end, the cyclosporine ophthalmic formulation was exposed to higher temperatures and times and PXRD data was taken from the precipitated part; it was found that the precipitate was close to the CsA form with characteristic XRD peaks at 2-theta (deg.) 6.9, 7.8, 9.4 and 15.9, as shown in Figure 4. Because this CsA form has lower solubility in water, its use may lead to a seeding effect either at the initial stage or during storage at higher temperatures, thus leading to batch failure.

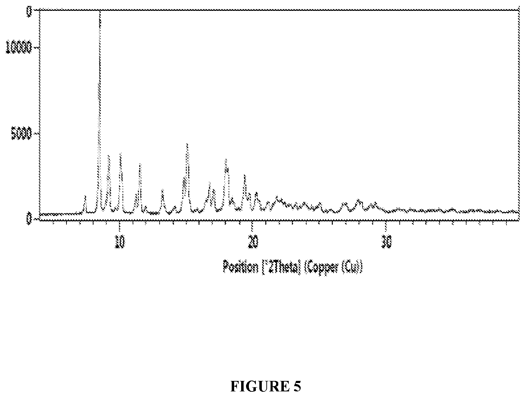

In another attempt, the behavior of CsA in Kolliphore RH 40 alone in absence of water is determined. The CsA was dissolved in Kolliphore RH 40 at 55°C and kept it for a longer time till the precipitation occurred. The precipitated part was separated out and PXRD was done. It was found that the cyclosporine has characteristic XRD peaks at 2-theta (deg.) 8.5, 9.3, 11.6 and 20.3, as shown in Figure 5. As a result, the process as disclosed in the present disclosure, in which there is reduced exposure of cyclosporine to, for example, KOLLIPHOR RH 40 at 55°C, can minimize this precipitation phenomenon which may be due to a possible conversion of the cyclosporine to the less soluble CsA form. Thus, the present disclosure provides for, among other things, improved storage stability.

### EXAMPLE 5

### Preparation method of the nanomicellar solution of Table 1

The polyoxyl 40 hydrogenated castor oil (Kolliphor RH40) was heated to about 50-60°C, until it liquefies, prior to introduction into the 10L glass vessel. The cyclosporine (CsA) was added while maintaining the vessel temperature at 55±2°C and dissolved by stirring at approximately 200-300 RPM for 75 minutes and visually inspected to ensure it is a clear solution with no visible particles. The temperature was gradually lowered to 35°C. Once it is completely dissolved, the temperature was increased to 55±2°C. The octoxynol-40 is then added. If the octoxynol-40 has solidified, it was heated at about 50-60°C until it liquefies prior to its addition.

A portion (approximately 90%) of the Water for Injection (WFI) was charged into the stainless-steel mixing tank and the temperature is maintained at 20-30°C throughout the process. While stirring, the API mixture was added at 55±2°C to the mixing tank and stirred for approximately 15 minutes while the remaining excipients were added in order of sodium phosphate monobasic, then sodium phosphate dibasic, then sodium chloride, and then polyvinylpyrrolidone.

After mixing for 15 minutes, the pH was checked and adjusted, if necessary, to 6.8± 0.2 using hydrochloric acid (1N) or sodium hydroxide (1N). The solution was adjusted to the final volume with WFI and filtered through 0.2 µm filter.

### EXAMPLE 6

### Preparation method of the nanomicellar solution of Table 1

The polyoxyl 40 hydrogenated castor oil (Kolliphor RH40) was heated to about 50-60°C, until it liquefies, prior to introduction into the 10L glass vessel. The cyclosporine (CsA) was added while maintaining the vessel temperature at 55±2°C for 20±2 minutes and then stirred at approximately 200-300 RPM for 15 minutes. The temperature was reduced gradually to 35°C under stirring and once it reaches the temperature 35°C, it is stirred for 60±5 minutes. The octoxynol-40 was then added. If the octoxynol-40 has solidified, it was heated at about 50-60°C until it liquefies prior to its addition.

A portion (approximately 90%) of the Water for Injection (WFI) was charged into the stainless-steel mixing tank and the temperature was maintained at 20-30°C throughout the process. While stirring, the CsA mixture was added to the mixing tank at 35±2°C and stirred for approximately 15 minutes while the remaining excipients were added in order of sodium phosphate monobasic, then sodium phosphate dibasic, then sodium chloride, and then polyvinylpyrrolidone.

After mixing for 15 minutes, the pH was checked and adjusted, if necessary, to 6.8± 0.2 using hydrochloric acid (1N) or sodium hydroxide (1N). The solution was adjusted to the final volume with WFI and filtered through 0.2 µm filter.

### EXAMPLE 7

### Preparation method of the nanomicellar solution of Table 1 - process under high speed

The polyoxyl 40 hydrogenated castor oil (Kolliphor RH40) was heated to about 50-60°C, until it liquefies, prior to introduction into the 10L glass vessel. The cyclosporine (CsA) was added while maintaining the vessel temperature at 55±2°C for 20±2 minutes and stirred at approximately >450 RPM for 15 minutes. The temperature was reduced gradually to 35°C under stirring and stirred for 60±5 minutes. The octoxynol-40 was then added. If the octoxynol-40 has solidified, it was heated at about 50-60°C until it liquefies prior to its addition.

A portion (approximately 90%) of the Water for Injection (WFI) was charged into the stainless-steel mixing tank and the temperature was maintained at 20-30°C throughout the process. While stirring, the CsA mixture was added to the mixing tank at 35±2°C and stirred for approximately 15 minutes while the remaining excipients were added in order of sodium phosphate monobasic, then sodium phosphate dibasic, then sodium chloride, and then polyvinylpyrrolidone.

After mixing for 15 minutes, the pH was checked and adjusted, if necessary, to 6.8± 0.2 using hydrochloric acid (1N) or sodium hydroxide (1N). The solution was adjusted to the final volume with WFI and filtered through 0.2 µm filter.

### EXAMPLE 8

### Preparation method of the nanomicellar solution of Table 1 - process at higher temperature

The polyoxyl 40 hydrogenated castor oil (Kolliphor RH40) is heated to about 50-60°C, until it liquefies, prior to introduction into the 10L glass vessel. The temperature was increased to 127-130°C. The cyclosporine (CsA) was added while maintaining the vessel temperature at 127-130°C and stirred at approximately 200-300 RPM for complete dissolution. The octoxynol-40 was then added. If the octoxynol-40 has solidified, it was heated at about 50-60°C until it liquefies prior to its addition.

A portion (approximately 90%) of the Water for Injection (WFI) was charged into the stainless-steel mixing tank and the temperature was maintained at 20-30°C throughout the process. While stirring, the CsA mixture was added at 127-130°C to the mixing tank and stirred for approximately 15 minutes while the remaining excipients are added in order of sodium phosphate monobasic, then sodium phosphate dibasic, then sodium chloride, and then polyvinylpyrrolidone.

After mixing for 15 minutes, the pH was checked and adjusted, if necessary, to 6. 8± 0.2 using hydrochloric acid (1N) or sodium hydroxide (1N). The solution was adjusted to the final volume with WFI and filtered through 0.2 µm filter.

### EXAMPLE 9

### Stability Studies

Nanomicellar ophthalmic formulation of Example 6 was tested after being stored at 25°C/40%RH for 6 months.

The formulation was tested for change in appearance, pH, osmolality, viscosity, Cyclosporine assay by HPLC method II, micelle size determination by Laser light scattering method and particulate matter presence.

| Tests | Specifications | Initial | 3M | 6M |
|---|---|---|---|---|
| Appearance | Clear colorless solution, essentially free from visible particulate matter | Complies | Complies | Complies |
| pH | 6.5-7.2 | 6.8 | 6.8 | 6.8 |
| Osmolality | 160-190mOsmol/Kg | 169 | 166 | 175 |
| Viscosity | 1.3 -2.0 cP | 1.4 | 1.4 | 1.4 |
| HPLC method II | | | | |
| Cyclosporine assay | 95.0 -105.0% of label claim | 97.8 | 99.2 | 98.2 |
| Total impurities | ≤2.0% area | 0.59 | 0.76 | 0.83 |
| Micelle size determination | Zavg: 13-16nm | 15 | 14 | 14 |
| | PdI ≤0.2 | 0.13 | 0.15 | 0.10 |
| Particulate matter (USP 788) | The average number of particles present in the units does not exceed: | | | |
| | Stage 1 : by method 1 - Light obscuration | | | |
| | 6000 per contained ≥10µm | NA | NA | NA |
| | 600 per container ≥25µm | NA | NA | NA |
| | Stage 2: method 2- Membrane microscopic | | | |
| | 6000 per contained ≥10µm | NA | NA | NA |
| | 600 per container ≥25µm | NA | NA | NA |
| Sterility | No evidence of microbial growth | Sterile | NA | NA |

As observed from the above data, the formulation is found to be both chemically and physically stable.

Even though certain specific embodiments are thoroughly described in the present application, it should be understood that the same concepts disclosed with respect to those specific embodiments are also applicable to other embodiments. Furthermore, individual elements of the formulations and methods disclosed herein are described with reference to particular embodiments only for the sake of convenience. It should be understood that individual elements of the formulations and methods disclosed herein are applicable to embodiments other than the specific embodiments in which they are described.

In addition, it should be understood that the scope of the present disclosure is not limited to the above-described embodiments, and those skilled in the art will appreciate that various modifications and alterations are possible without departing from the scope of the present disclosure. For example, the batch sizes may be altered by a person having ordinary skill in the art while staying within the present disclosure.

## Claims

1. A method of making a stable nanomicellar ophthalmic formulation comprising:
0.09 wt % cyclosporine,
about 1.0 wt % hydrogenated 40 polyoxyl castor oil,
about 0.05 wt % octoxynol-40,
about 0.20-0.550 wt % sodium phosphate monobasic,
about 0.23-0.465 wt % sodium phosphate dibasic,
about 0.05 wt % sodium chloride,
about 0.3 wt % povidone,
sodium hydroxide/hydrochloric acid, and
water for injection,
wherein the method comprises the steps of:
a) mixing the cyclosporine with the hydrogenated 40 polyoxyl castor oil at a temperature of 55°C ± 2°C or above to form a mixture A;
b) lowering the temperature of the mixture A to a temperature of 35°C ± 2°C prior to complete dissolution of the cyclosporine, and adding the octoxynol-40 to form an API mixture;
c) adding the API mixture to Water for Injection (WFI);
d) adding to the result of step (c) in the following order: the sodium phosphate monobasic, then the sodium phosphate dibasic, then the sodium chloride, and then the povidone; and,
e) adjusting pH of the result of step (d) to 6.5 to 7.2 with the sodium hydroxide/hydrochloric acid, and bringing to the final volume with WFI.

2. The method of claim 1, wherein the cyclosporine in step (a) is mixed at 200-300 RPM.

3. The method of claim 1, wherein the temperature of the mixture A in step (b) is lowered to 35°C ± 2°C in 40-50 minutes.

4. The method of claim 1, wherein step (b) further comprises stirring the mixture A for 60 ± 5 minutes at a temperature of 35°C ± 2°C.

5. A stable nanomicellar ophthalmic formulation prepared by the method as described in any of the preceding claims, wherein the formulation is substantially free of (i) a cyclosporine form with characteristic XRD peaks at 2-theta (deg.) 7.4, 8.7, 14.4 and 17.5 or (ii) a cyclosporine form with characteristic XRD peaks at 2-theta (deg.) 8.5, 9.3, 11.6 and 20.3.

6. The stable nanomicellar ophthalmic formulation of claim 5, wherein the osmolality of the formulation is between about 150 to about 200 mOsmol/kg.

7. The stable nanomicellar ophthalmic formulation of claim 6, wherein the osmolality of the formulation is about 160 to about 190 mOsmol/kg.

8. The stable nanomicellar ophthalmic formulation of claim 5, wherein the formulation comprises cyclosporine form with characteristic XRD peaks at 2-theta (deg.) 6.9, 7.8, 9.4 and 15.9.

9. The stable nanomicellar ophthalmic formulation of claim 5, wherein the formulation comprises an amorphous form of cyclosporine.

10. A stable nanomicellar ophthalmic formulation comprising:
cyclosporine, hydrogenated 40 polyoxyl castor oil, octoxynol-40, and an aqueous vehicle, wherein the formulation is made by a method comprising:
a) mixing the cyclosporine with the hydrogenated 40 polyoxyl castor oil at a temperature of 127-130°C until completely dissolved to form a mixture A;
b) adding the octoxynol-40 to the mixture A at 127-130°C; and
c) mixing the resulting mixture of (b) with the aqueous vehicle at a temperature of at 127-130°C,
wherein the cyclosporine is (i) a cyclosporine form having characteristic XRD peaks at 2-theta (deg.) 7.4, 8.7, 14.4 and 17.5; or (ii) a cyclosporine form having characteristic XRD peaks at 2-theta (deg.) 8.5, 9.3, 11.6 and 20.3.

11. The stable nanomicellar ophthalmic formulation of claim 10, comprising:
0.09 wt % cyclosporine,
about 1.0 wt % hydrogenated 40 polyoxyl castor oil, and
about 0.05 wt % octoxynol-40.

12. A method of making a stable nanomicellar ophthalmic formulation comprising cyclosporine, hydrogenated 40 polyoxyl castor oil, octoxynol-40, and an aqueous vehicle, the method comprising:
a) mixing the cyclosporine with the hydrogenated 40 polyoxyl castor oil at a temperature of 127-130°C until completely dissolved to form a mixture A;
b) adding the octoxynol-40 to the mixture A at 127-130°C; and
c) mixing the resulting mixture of (b) with the aqueous vehicle at a temperature of at 127-130°C.

13. The method of claim 12, wherein the stable nanomicellar ophthalmic formulation comprises:
0.09 wt % cyclosporine,
about 1.0 wt % hydrogenated 40 polyoxyl castor oil, and
about 0.05 wt % octoxynol-40.

14. The method of claim 12 or 13, wherein the cyclosporine is (i) a cyclosporine form having characteristic XRD peaks at 2-theta (deg.) 7.4, 8.7, 14.4 and 17.5; or (ii) a cyclosporine form having characteristic XRD peaks at 2-theta (deg.) 8.5, 9.3, 11.6 and 20.3.

## Patentansprüche

1. Verfahren zur Herstellung einer stabilen nanomizellaren ophthalmischen Formulierung, die Folgendes umfasst:
0,09 Gew.-% Cyclosporin,
etwa 1,0 Gew.-% hydriertes 40-Polyoxyl-Rizinusöl,
etwa 0,05 Gew.-% Octoxynol-40,
etwa 0,20-0,550 Gew.-% einbasiges Natriumphosphat,
etwa 0,23-0,465 Gew.-% dibasisches Natriumphosphat,
etwa 0,05 Gew.-% Natriumchlorid,
etwa 0,3 Gew.-% Povidon,
Natriumhydroxid/Salzsäure, und
Wasser für Injektionszwecke,
wobei das Verfahren die folgenden Schritte umfasst:
a) Mischen des Cyclosporins mit dem hydrierten 40-Polyoxyl-Rizinusöl bei einer Temperatur von 55 °C ± 2 °C oder höher, um eine Mischung A zu bilden;
b) Senken der Temperatur der Mischung A auf eine Temperatur von 35 °C ± 2 °C vor der vollständigen Auflösung des Cyclosporins und Hinzufügen des Octoxynol-40, um eine API-Mischung zu bilden;
c) Hinzufügen der API-Mischung zu Wasser für Injektionszwecke (WFI);
d) Hinzufügen zum Ergebnis aus Schritt (c) in der folgenden Reihenfolge: das einbasige Natriumphosphat, dann das dibasische Natriumphosphat, dann das Natriumchlorid und dann das Povidon; und,
e) Einstellen des pH-Werts des Ergebnisses aus Schritt (d) auf 6,5 bis 7,2 mit Natriumhydroxid/Salzsäure und Auffüllen auf das Endvolumen mit WFI.

2. Verfahren nach Anspruch 1, wobei das Cyclosporin in Schritt (a) bei 200-300 U/min gemischt wird.

3. Verfahren nach Anspruch 1, wobei die Temperatur der Mischung A in Schritt (b) in 40-50 Minuten auf 35 °C ± 2 °C gesenkt wird.

4. Verfahren nach Anspruch 1, wobei Schritt (b) ferner das Rühren der Mischung A für 60 ± 5 Minuten bei einer Temperatur von 35 °C ± 2 °C umfasst.

5. Stabile nanomizellare ophthalmische Formulierung, die nach dem in einem der vorhergehenden Ansprüche beschriebenen Verfahren vorbereitet wird, wobei die Formulierung im Wesentlichen frei von (i) einer Cyclosporin-Form mit charakteristischen XRD-Peaks bei 2-Theta (Grad) 7,4, 8,7, 14,4 und 17,5 oder (ii) einer Cyclosporin-Form mit charakteristischen XRD-Peaks bei 2-Theta (Grad) 8,5, 9,3, 11,6 und 20,3 ist.

6. Stabile nanomizellare ophthalmische Formulierung nach Anspruch 5, wobei die Osmolalität der Formulierung zwischen etwa 150 und etwa 200 mOsmol/kg liegt.

7. Stabile nanomizellare ophthalmische Formulierung nach Anspruch 6, wobei die Osmolalität der Formulierung etwa 160 bis etwa 190 mOsmol/kg beträgt.

8. Stabile nanomizellare ophthalmische Formulierung nach Anspruch 5, wobei die Formulierung eine Cyclosporin-Form mit charakteristischen XRD-Peaks bei 2-Theta (Grad) 6,9, 7,8, 9,4 und 15,9 umfasst.

9. Stabile nanomizellare ophthalmische Formulierung nach Anspruch 5, wobei die Formulierung eine amorphe Form von Cyclosporin umfasst.

10. Stabile nanomizellare ophthalmische Formulierung, die Folgendes umfasst:
Cyclosporin, hydriertes 40-Polyoxyl-Rizinusöl, Octoxynol-40 und einen wässrigen Träger, wobei die Formulierung nach einem Verfahren hergestellt wird, das Folgendes umfasst:
a) Mischen des Cyclosporins mit dem hydrierten 40-Polyoxyl-Rizinusöl bei einer Temperatur von 127-130 °C bis zur vollständigen Auflösung, um eine Mischung A zu bilden;
b) Hinzufügen des Octoxynol-40 zu der Mischung A bei 127-130 °C; und
c) Mischen der resultierenden Mischung aus (b) mit dem wässrigen Träger bei einer Temperatur von 127-130 °C,
wobei das Cyclosporin (i) eine Cyclosporin-Form ist, die charakteristische XRD-Peaks bei 2-Theta (Grad) 7,4, 8,7, 14,4 und 17,5 aufweist; oder (ii) eine Cyclosporin-Form, die charakteristische XRD-Peaks bei 2-Theta (Grad) 8,5, 9,3, 11,6 und 20,3 aufweist.

11. Stabile nanomizellare ophthalmische Formulierung nach Anspruch 10, die Folgendes umfasst:
0,09 Gew.-% Cyclosporin,
etwa 1,0 Gew.-% hydriertes 40-Polyoxyl-Rizinusöl, und
etwa 0,05 Gew.-% Octoxynol-40.

12. Verfahren zur Herstellung einer stabilen nanomizellaren ophthalmischen Formulierung, die Cyclosporin, hydriertes 40-Polyoxyl-Rizinusöl, Octoxynol-40 und einen wässrigen Träger umfasst, wobei das Verfahren Folgendes umfasst:
a) Mischen des Cyclosporins mit dem hydrierten 40-Polyoxyl-Rizinusöl bei einer Temperatur von 127-130 °C bis zur vollständigen Auflösung, um eine Mischung A zu bilden;
b) Hinzufügen des Octoxynol-40 zu der Mischung A bei 127-130 °C; und
c) Mischen der resultierenden Mischung aus (b) mit dem wässrigen Träger bei einer Temperatur von 127-130 °C.

13. Verfahren nach Anspruch 12, wobei die stabile nanomizellare ophthalmische Formulierung Folgendes umfasst:
0,09 Gew.-% Cyclosporin,
etwa 1,0 Gew.-% hydriertes 40-Polyoxyl-Rizinusöl, und
etwa 0,05 Gew.-% Octoxynol-40.

14. Verfahren nach Anspruch 12 oder 13, wobei das Cyclosporin (i) eine Cyclosporin-Form ist, die charakteristische XRD-Peaks bei 2-Theta (Grad) 7,4, 8,7, 14,4 und 17,5 aufweist; oder (ii) eine Cyclosporin-Form, die charakteristische XRD-Peaks bei 2-Theta (Grad) 8,5, 9,3, 11,6 und 20,3 aufweist.

## Revendications

1. Procédé de préparation d'une formulation ophtalmique nanomicellaire stable comprenant :
0,09 % en poids de cyclosporine,
environ 1,0 % en poids d'huile de ricin polyoxyl 40 hydrogénée,
environ 0,05 % en poids d'octoxynol-40,
environ 0,20 à 0,550 % en poids de phosphate de sodium monobasique,
environ 0,23 à 0,465 % en poids de phosphate de sodium dibasique,
environ 0,05 % en poids de chlorure de sodium,
environ 0,3 % en poids de povidone,
de l'hydroxyde de sodium/acide chlorhydrique, et
de l'eau pour préparation injectable,
où le procédé comprend les étapes consistant à :
a) mélanger la cyclosporine avec l'huile de ricin polyoxyl 40 hydrogénée à une température de 55 °C ± 2 °C ou supérieure pour former un mélange A ;
b) abaisser la température du mélange A à une température de 35 °C ± 2 °C avant la dissolution complète de la cyclosporine, et ajouter l'octoxynol-40 pour former un mélange de principe actif pharmaceutique API ;
c) ajouter le mélange d'API à de l'eau pour préparation injectable (WFI) ;
d) ajouter au résultat de l'étape (c) dans l'ordre suivant : le phosphate de sodium monobasique, puis le phosphate de sodium dibasique, puis le chlorure de sodium, puis la povidone ; et,
e) ajuster le pH du résultat de l'étape (d) de 6,5 à 7,2 avec l'hydroxyde de sodium/acide chlorhydrique, et compléter au volume final avec de la WFI.

2. Procédé selon la revendication 1, où la cyclosporine à l'étape (a) est mélangée à une vitesse de 200 à 300 tr/min (RPM).

3. Procédé selon la revendication 1, où la température du mélange A à l'étape (b) est abaissée à 35 °C ± 2 °C en 40 à 50 minutes.

4. Procédé selon la revendication 1, où l'étape (b) comprend en outre l'agitation du mélange A pendant 60 ± 5 minutes à une température de 35 °C ± 2 °C.

5. Formulation ophtalmique nanomicellaire stable préparée par le procédé tel que décrit selon l'une quelconque des revendications précédentes, où la formulation est sensiblement exempte de (i) une forme de cyclosporine présentant des pics caractéristiques de diffraction des rayons X (DRX) à 2-thêta (degrés) de 7,4, 8,7, 14,4 et 17,5 ou (ii) une forme de cyclosporine présentant des pics caractéristiques de DRX à 2-thêta (degrés) de 8,5, 9,3, 11,6 et 20,3.

6. Formulation ophtalmique nanomicellaire stable selon la revendication 5, où l'osmolalité de la formulation est comprise entre environ 150 et environ 200 mOsmol/kg.

7. Formulation ophtalmique nanomicellaire stable selon la revendication 6, où l'osmolalité de la formulation est d'environ 160 à environ 190 mOsmol/kg.

8. Formulation ophtalmique nanomicellaire stable selon la revendication 5, où la formulation comprend une forme de cyclosporine présentant des pics caractéristiques de DRX à 2-thêta (degrés) de 6,9, 7,8, 9,4 et 15,9.

9. Formulation ophtalmique nanomicellaire stable selon la revendication 5, où la formulation comprend une forme amorphe de cyclosporine.

10. Formulation ophtalmique nanomicellaire stable comprenant :
de la cyclosporine, de l'huile de ricin polyoxyl 40 hydrogénée, de l'octoxynol-40, et un véhicule aqueux, où la formulation est préparée selon un procédé comprenant les étapes consistant à :
a) mélanger la cyclosporine avec l'huile de ricin polyoxyl 40 hydrogénée à une température de 127 à 130 °C jusqu'à dissolution complète pour former un mélange A ;
b) ajouter l'octoxynol-40 au mélange A à une température de 127 à 130 °C ; et
c) mélanger le mélange résultant de l'étape (b) avec le véhicule aqueux à une température de 127 à 130 °C,
où la cyclosporine est (i) une forme de cyclosporine ayant des pics caractéristiques de DRX à 2-thêta (degrés) de 7,4, 8,7, 14,4 et 17,5 ; ou (ii) une forme de cyclosporine ayant des pics caractéristiques de DRX à 2-thêta (degrés) de 8,5, 9,3, 11,6 et 20,3.

11. Formulation ophtalmique nanomicellaire stable selon la revendication 10, comprenant :
0,09 % en poids de cyclosporine,
environ 1,0 % en poids d'huile de ricin polyoxyl 40 hydrogénée, et
environ 0,05 % en poids d'octoxynol-40.

12. Procédé de préparation d'une formulation ophtalmique nanomicellaire stable comprenant :
de la cyclosporine, de l'huile de ricin polyoxyl 40 hydrogénée, de l'octoxynol-40, et un véhicule aqueux, le procédé comprenant les étapes consistant à :
a) mélanger la cyclosporine avec l'huile de ricin polyoxyl 40 hydrogénée à une température de 127 à 130 °C jusqu'à dissolution complète pour former un mélange A ;
b) ajouter l'octoxynol-40 au mélange A à une température de 127 à 130 °C ; et
c) mélanger le mélange résultant de l'étape (b) avec le véhicule aqueux à une température de 127 à 130 °C.

13. Procédé selon la revendication 12, où la formulation ophtalmique nanomicellaire stable comprend :
0,09 % en poids de cyclosporine,
environ 1,0 % en poids d'huile de ricin polyoxyl 40 hydrogénée, et
environ 0,05 % en poids d'octoxynol-40.

14. Procédé selon la revendication 12 ou 13, où la cyclosporine est (i) une forme de cyclosporine ayant des pics caractéristiques de DRX à 2-thêta (degrés) de 7,4, 8,7, 14,4 et 17,5 ; ou (ii) une forme de cyclosporine ayant des pics caractéristiques de DRX à 2-thêta (degrés) de 8,5, 9,3, 11,6 et 20,3.
